# EUROPEAN PATENT APPLICATION

(11) **EP 3 135 376 A1**
(43) Date of publication of application: **01.03.2017**
(21) Application number: 16185474.0
(22) Date of filing: 24.08.2016
(51) Int. Cl.: B01L 3/00, B01L 7/00

(54) **NUCLEIC ACID AMPLIFICATION REACTION CONTAINER, NUCLEIC ACID AMPLIFICATION REACTION DEVICE, AND REACTION METHOD FOR AMPLIFYING NUCLEIC ACID**

(30) Priority: 26.08.2015 JP 2015167150
(71) Applicant: Seiko Epson Corporation, Tokyo 160-8801 (JP)
(72) Inventor: Takagi, Fumio, Nagano, 392-8502 (JP); Murayama, Toshiro, Nagano, 392-8502 (JP)
(74) Representative: Hoffmann Eitle

(57) **Abstract**

A nucleic acid amplification reaction container includes: a flow path which is formed between a first inner wall surface and a second inner wall surface that opposes the first inner wall surface, and has the longitudinal direction in the direction along the first inner wall surface, in which the distance between the first inner wall surface and the second inner wall surface is the distance by which nucleic acid amplification reaction liquid comes into contact with both of the first inner wall surface and the second inner wall surface in a case where the nucleic acid amplification reaction liquid is guided therein, and in which, in the section which is orthogonal to the longitudinal direction of the flow path, the length in the direction parallel to the first inner wall surface is 5 times or more the distance between the first inner wall surface and the second inner wall surface.

## Description

### BACKGROUND

### 1. Technical Field

The present invention relates to a nucleic acid amplification reaction container, a nucleic acid amplification reaction device, and a reaction method for amplifying nucleic acid.

### 2. Related Art

As a method of amplifying nucleic acid at high speed, a method of generating a heat cycle to the temperature of nucleic acid amplification reaction liquid at high speed by putting oil and a small amount of the nucleic acid amplification reaction liquid into a cylindrical nucleic acid amplification reaction container, maintaining one end of the container at high temperature and the other end at low temperature, rotating the container, and alternately switching a state where one end thereof becomes a lower part in the vertical direction and the reaction liquid is positioned in the oil having high temperature, and a state where the other end becomes a lower part in the vertical direction and the reaction liquid is positioned in the oil having low temperature is known (for example, refer to JP-A-2012-115208).

In a lifting type PCR device which moves the reaction liquid by an action of gravity according to JP-A-2012-115208, the cylindrical nucleic acid amplification reaction container is used, and the shape of the section of a flow path in the reaction container is circular. In addition, by making the diameter of a liquid droplet of the nucleic acid amplification reaction liquid smaller than the diameter of the flow path of the reaction container, the movement of the liquid droplet is realized.

However, in this configuration, the liquid droplet and a wall surface of the flow path come into point contact with each other or come into line contact with each other, and an area in which the liquid droplet comes into contact with the wall surface of the flow path is small. Therefore, the efficiency of heat conduction in a case where the heat is directly transmitted to the liquid droplet from the wall surface is not necessarily excellent. In other words, in the technology of JP-A-2012-115208, the heat of a side wall of the container heated by a heater of a reaction device is mainly conducted to the liquid droplet via the oil, and the contact area between the side wall and the liquid droplet is not sufficient for directly conducting the heat from the side wall of the container to the liquid droplet.

In addition, in the technology of JP-A-2012-115208, since the inside of the nucleic acid amplification reaction container is filled with the nucleic acid amplification reaction liquid and the oil, and the oil flows in a case where the nucleic acid amplification reaction liquid is moved in the oil, flow resistance of the oil is generated. Since the oil has constant viscosity, the moving speed of the nucleic acid amplification reaction liquid is restricted due to the presence of the oil. Therefore, there is room for improvement from the viewpoint of accelerating the nucleic acid amplification reaction.

### SUMMARY

An advantage of some aspects of the invention is to provide a nucleic acid amplification reaction container, a nucleic acid amplification reaction device, and a reaction method for amplifying nucleic acid, which can perform amplification of nucleic acid at high speed.

The invention can be implemented as the following aspects or application examples.

A nucleic acid amplification reaction container according to an aspect of the invention includes: a flow path which is formed between a first inner wall surface and a second inner wall surface that opposes the first inner wall surface, and has the longitudinal direction in a case of being seen from the direction perpendicular to the first inner wall surface, in which the distance between the first inner wall surface and the second inner wall surface is the distance by which the nucleic acid amplification reaction liquid comes into contact with both of the first inner wall surface and the second inner wall surface in a case where the nucleic acid amplification reaction liquid is guided therein, and in which, in the section of the flow path which is orthogonal to the longitudinal direction, the length in the direction parallel to the first inner wall surface is 5 times or more the distance between the first inner wall surface and the second inner wall surface.

According to the nucleic acid amplification reaction container, in a case where the nucleic acid amplification reaction liquid is guided therein, the nucleic acid amplification reaction liquid comes into contact with both of the first inner wall surface and the second inner wall surface, and the contact state becomes surface contact. In addition, in the section of the flow path which is orthogonal to the longitudinal direction, as the section in which the length in the direction parallel to the first inner wall surface is 5 times or more the distance between the first inner wall surface and the second inner wall surface is provided, the contact area can increase. Therefore, direct heat exchange between the nucleic acid amplification reaction liquid and the inner wall surface is excellently performed, and the nucleic acid amplification reaction liquid can achieve the predetermined temperature faster. Accordingly, the amplification of the nucleic acid can be performed at high speed.

A nucleic acid amplification reaction container according to another aspect of the invention includes: a flow path which is formed between a first inner wall surface and a second inner wall surface that opposes the first inner wall surface, and has the longitudinal direction in a case of being seen from the direction perpendicular to the first inner wall surface, in which the distance between the first inner wall surface and the second inner wall surface is the distance by which nucleic acid amplification reaction liquid comes into contact with both of the first inner wall surface and the second inner wall surface in a case where the nucleic acid amplification reaction liquid is guided therein, and in which the nucleic acid amplification reaction liquid and gas are disposed in the flow path.

According to the nucleic acid amplification reaction container, in a case where the nucleic acid amplification reaction liquid is guided therein, the nucleic acid amplification reaction liquid comes into contact with both of the first inner wall surface and the second inner wall surface, and the contact state becomes surface contact. Therefore, direct heat exchange between the nucleic acid amplification reaction liquid and the inner wall surface is excellently performed, and the nucleic acid amplification reaction liquid can achieve the predetermined temperature faster. Furthermore, as the gas is guided into the flow path, it becomes easy for the nucleic acid amplification reaction liquid to move in the flow path, and the nucleic acid amplification reaction liquid can be more rapidly moved to the predetermined position. Accordingly, the nucleic acid amplification reaction liquid can achieve the predetermined temperature much earlier. Accordingly, the amplification of the nucleic acid can be performed at high speed.

In the nucleic acid amplification reaction container according to the aspect of the invention, oil may be disposed in the flow path.

According to this configuration, in the flow path, since the oil is positioned between the nucleic acid amplification reaction liquid and the gas, evaporation of components of the nucleic acid amplification reaction liquid is suppressed, and the nucleic acid can be more accurately amplified. In addition, even when the oil is present, since the gas is present, deterioration of the moving speed of the nucleic acid amplification reaction liquid is suppressed.

In the nucleic acid amplification reaction container according to the aspect of the invention, the volume of the gas may be 50% or more of the capacity of the flow path.

According to this configuration, for example, even when each half of the container is set to have a temperature different from each other, the entire nucleic acid amplification reaction liquid is likely to achieve each temperature.

In the nucleic acid amplification reaction container according to the aspect of the invention, the distance between the first inner wall surface and the second inner wall surface may be from 0.2 mm to 3.0 mm.

According to the nucleic acid amplification reaction container with this configuration, the nucleic acid amplification reaction liquid can more reliably come into contact with the first inner wall surface and the second inner wall surface. In addition, the size of the nucleic acid amplification reaction container does not become extremely large, and for example, workability can be improved.

In the nucleic acid amplification reaction container according to the aspect of the invention, at least one of a first wall which forms the first inner wall surface and a second wall which forms the second inner wall surface may have a thickness of 0.01 mm to 0.5 mm.

According to the nucleic acid amplification reaction container with this configuration, it is possible to reduce the distance of heat conduction of the container, and to reduce a difference in temperature between the outer side and the inner side. Therefore, for example, the nucleic acid amplification reaction liquid can achieve the set temperature of the external heater faster.

In the nucleic acid amplification reaction container according to the aspect of the invention, at least one of the first wall and the second wall may be deformed in a case where inner pressure of the flow path increases.

According to the nucleic acid amplification reaction container with this configuration, for example, in a case where the nucleic acid amplification reaction container is inserted into a heat block having a hole in an external shape of the nucleic acid amplification reaction container, and in a case where pressure of the inside of the container is raised by the heat of the heat block, it is possible to press the first wall and the second wall to the heat block. Accordingly, heat exchange between the outside of the container and the inside of the container can be more efficiently performed.

In the nucleic acid amplification reaction container according to the aspect of the invention, at least one of walls which form the flow path may have light transmission properties.

According to the nucleic acid amplification reaction container with this configuration, it is possible to irradiate the nucleic acid amplification reaction liquid with light from the outside, and to easily observe fluorescence which is generated in a case where a fluorescent probe or the like is included in the nucleic acid amplification reaction liquid.

In the nucleic acid amplification reaction container according to the aspect of the invention, the volume of the nucleic acid amplification reaction liquid may be 1 µl to 100 µl.

According to the nucleic acid amplification reaction container with this configuration, the nucleic acid amplification reaction liquid can more reliably come into contact with the first inner wall surface and the second inner wall surface. In addition, the size of the nucleic acid amplification reaction container does not become extremely large, and for example, workability can be improved.

In the nucleic acid amplification reaction container according to the aspect of the invention, the first inner wall surface and the second inner wall surface may be hydrophobic.

According to the nucleic acid amplification reaction container with this configuration, it is difficult to wet the first inner wall surface and the second inner wall surface with the nucleic acid amplification reaction liquid, and it is easier to move the nucleic acid amplification reaction liquid in the flow path. Accordingly, the nucleic acid amplification reaction liquid can move at a higher speed.

The nucleic acid amplification reaction container according to the aspect of the invention may further include a lid body which seals the flow path, and the lid body may have a shape including a cylindrical surface which considers the longitudinal direction as a shaft.

According to the nucleic acid amplification reaction container with this configuration, it is easy for a user to handle the nucleic acid amplification reaction container, and for example, workability can be improved.

A nucleic acid amplification reaction device according to still another aspect of the invention includes: the nucleic acid amplification reaction container described above; a mounting portion which mounts the nucleic acid amplification reaction container; a first heating portion which heats a first region of the nucleic acid amplification reaction container; a second heating portion which heats a second region; and a driving mechanism which switches disposition of the mounting portion, the first region, and the second region between a first disposition and a second disposition, in which the first heating portion heats the first region to a first temperature and the second heating portion heats the second region to a second temperature different from the first temperature, in which the first disposition is a disposition in which the first region is below the second region with respect to the direction in which gravity acts, and the second disposition is a disposition in which the second region is below the first region with respect to the direction in which gravity acts.

According to the nucleic acid amplification reaction device, the nucleic acid can be amplified at high speed.

A reaction method for amplifying nucleic acid according to yet another aspect of the invention includes: guiding the nucleic acid amplification reaction liquid into the above-described nucleic acid amplification reaction container, and disposing at least the nucleic acid amplification reaction liquid and gas in the flow path; heating a first region of the nucleic acid amplification reaction container to a first temperature; heating a second region of the nucleic acid amplification reaction container to a second temperature different from the first temperature; moving the nucleic acid amplification reaction liquid in the flow path, and moving the nucleic acid amplification reaction liquid from the first region to the second region; and moving the nucleic acid amplification reaction liquid in the flow path, and moving the nucleic acid amplification reaction liquid from the second region to the first region.

According to the reaction method for amplifying nucleic acid, the nucleic acid can be amplified at high speed.

### BRIEF DESCRIPTION OF THE DRAWINGS

The invention will be described with reference to the accompanying drawings, wherein like numbers reference like elements.
Figs. 1A and 1B are perspective views of a nucleic acid amplification reaction device according to an embodiment. Fig. 1A illustrates a state where a lid is closed, and Fig. 1B illustrates a state where the lid is open.
Fig. 2 is an exploded perspective view of a main body in the nucleic acid amplification reaction device according to the embodiment.
Figs. 3A and 3B are sectional views schematically illustrating sections taken along line A-A of Fig. 1A of the main body in the nucleic acid amplification reaction device according to the embodiment. Fig. 3A illustrates a first disposition, and Fig. 3B illustrates a second disposition.
Figs. 4A and 4B are schematic views of a section of a nucleic acid amplification reaction container according to the embodiment. Fig. 4A is a section cut by a surface parallel to a first inner wall surface and a second inner wall surface which oppose each other in the reaction container, and Fig. 4B is a section cut by a surface orthogonal to the first inner wall surface and the second inner wall surface of the reaction container.

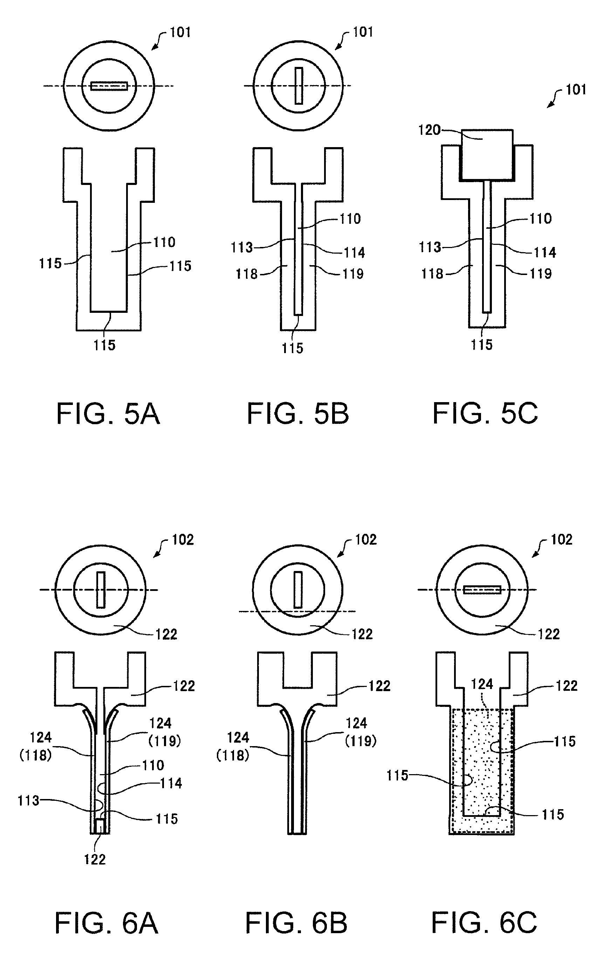

Figs. 5A to 5C are schematic views of the section of the nucleic acid amplification reaction container according to the embodiment. Fig. 5A is a section cut by the surface parallel to the first inner wall surface and the second inner wall surface which oppose each other in the reaction container, and Fig. 5B is a section cut by the surface orthogonal to the first inner wall surface and the second inner wall surface in the reaction container. Fig. 5C is a section cut by the surface orthogonal to the first inner wall surface and the second inner wall surface of the reaction container including a lid body.
Figs. 6A to 6C are schematic views of the section of the nucleic acid amplification reaction container according to the embodiment. Figs. 6A and 6B are sections cut by the surface orthogonal to the first inner wall surface and the second inner wall surface of the reaction container. Fig. 6C is a section cut by the surface parallel to the first inner wall surface and the second inner wall surface of the reaction container including the lid body.
Fig. 7 is a perspective view schematically illustrating the nucleic acid amplification reaction container according to the embodiment.
Fig. 8 is a schematic view of a section of the nucleic acid amplification reaction container according to the embodiment.
Fig. 9 is a flow chart illustrating processing in which the nucleic acid amplification reaction device according to the embodiment is used.
Figs. 10A and 10B are perspective views schematically illustrating the nucleic acid amplification reaction device according to a modification example. Fig. 10A illustrates a state where the lid is closed, and Fig. 10B illustrates a state where the lid is open.
Fig. 11 is a schematic view of a section taken along line B-B of Fig. 10A of the main body in a nucleic acid amplification reaction device according to the modification example.
Fig. 12 is a perspective view schematically illustrating the nucleic acid amplification reaction device according to the modification example.
Fig. 13 is a perspective view schematically illustrating the nucleic acid amplification reaction device according to the modification example.
Fig. 14 is a graph illustrating a result of an experiment example.
Fig. 15 is a graph illustrating a result of an experiment example.

### DESCRIPTION OF EXEMPLARY EMBODIMENTS

Hereinafter, several embodiments of the invention will be described. The embodiments described below describe an example of the invention. The invention is not limited to the following embodiments, and includes various modification aspects which are realized within a range of the invention without changing the main idea of the invention. In addition, all of the configurations described below are not necessarily configurations of the invention.

### 1. Nucleic Acid Amplification Reaction Device

### 1.1. Configuration of Nucleic Acid Amplification Reaction Device

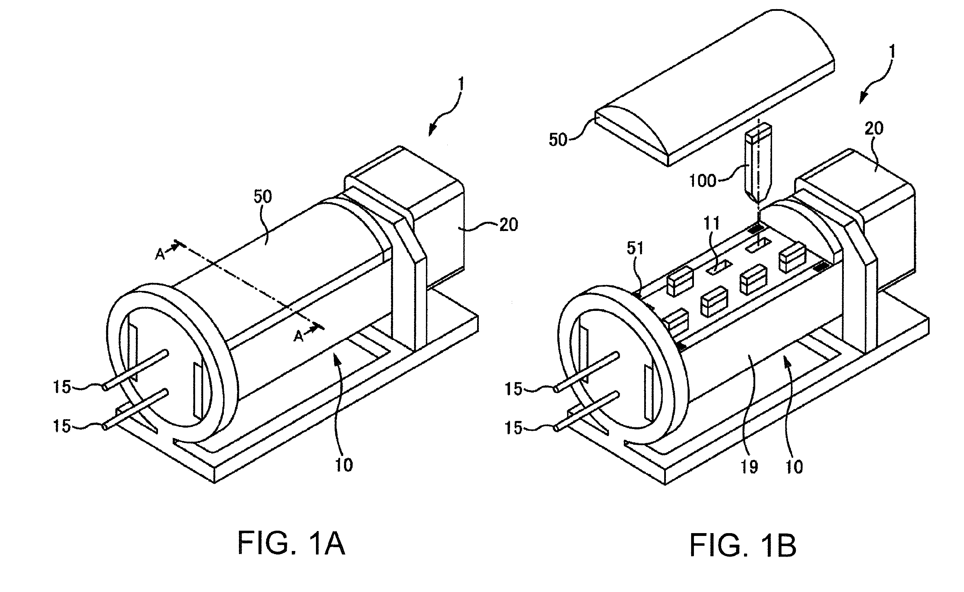

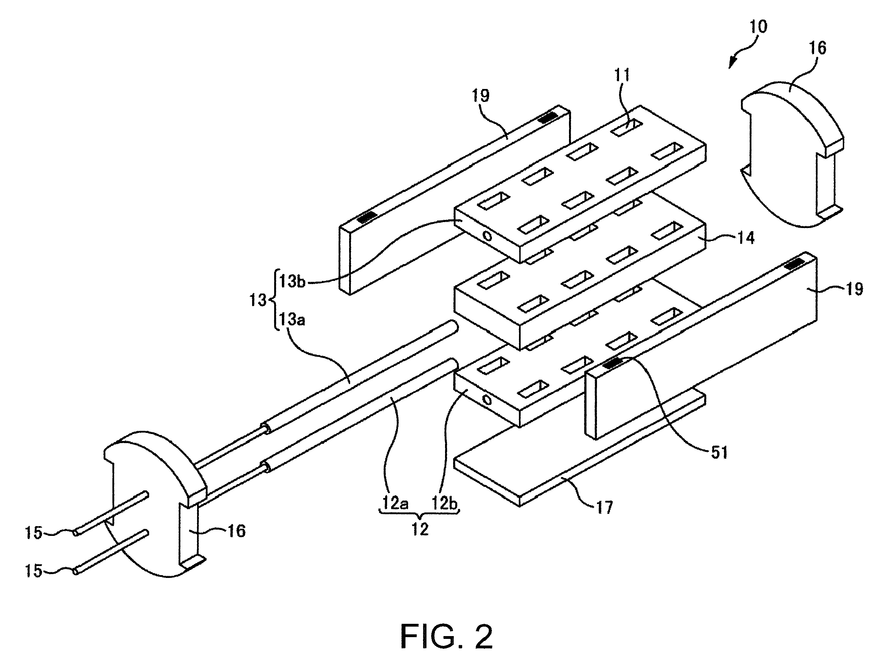

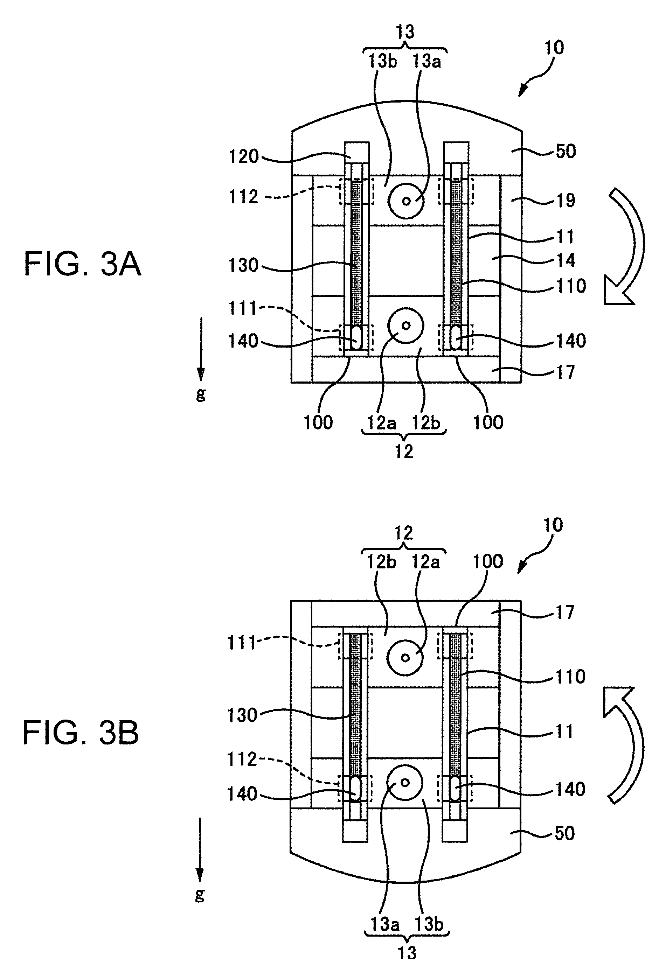

Figs. 1A and 1B are perspective views of a nucleic acid amplification reaction device 1 according to the embodiment. Fig. 1A illustrates a state where a lid 50 of the nucleic acid amplification reaction device 1 is closed, and Fig. 1B illustrates a state where the lid 50 of the nucleic acid amplification reaction device 1 is open, and a state where a nucleic acid amplification reaction container is mounted on a mounting portion 11. Fig. 2 is an exploded perspective view of a main body 10 in the nucleic acid amplification reaction device 1 according to the embodiment. Figs. 3A and 3B are sectional views schematically illustrating sections taken along line A-A of Fig. 1A of the main body 10 in the nucleic acid amplification reaction device according to the embodiment. In Figs. 1A to 3B, a reaction container 100 of a first aspect which will be described later is illustrated as an example of the reaction container 100.

As illustrated in Fig. 1A, the nucleic acid amplification reaction device 1 according to the embodiment includes the main body 10 and a driving mechanism 20. In addition, as illustrated in Fig. 2, the main body 10 includes the mounting portion 11, a first heating portion 12 (corresponding to a heating portion), and a second heating portion 13. A spacer 14 is provided between the first heating portion 12 and the second heating portion 13. In the main body 10 of the embodiment, the first heating portion 12 is disposed on a bottom plate 17 side, and the second heating portion 13 is disposed on the lid 50 side. In the main body 10 of the embodiment, the first heating portion 12, the second heating portion 13, and the spacer 14 are fixed to a flange 16, the bottom plate 17, and a fixing plate 19.

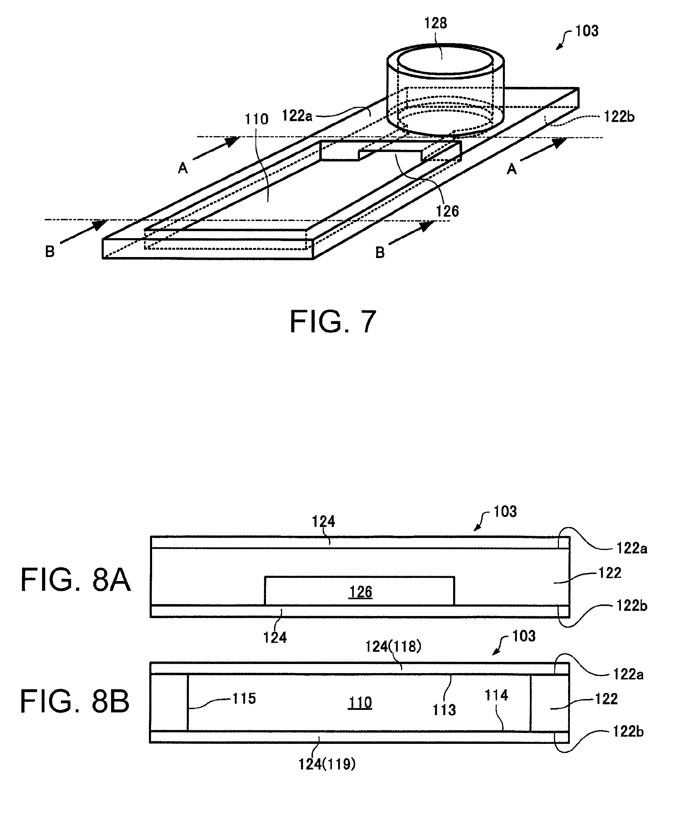

The mounting portion 11 has a structure in which the reaction container 100 which will be described later is mounted. As illustrated in Figs. 1B and 2, the mounting portion 11 of the embodiment has a slot structure in which the reaction container 100 is mounted being inserted, and has a structure in which the reaction container 100 is inserted into a hole which penetrates a first heat block 12b of the first heating portion 12 (heating portion), the spacer 14, and a second heat block 13b of the second heating portion 13. The number of mounting portions 11 may be plural, and in the example of Fig. 1B, 8 mounting portions 11 are provided in the main body 10.

It is preferable that the nucleic acid amplification reaction device 1 of the embodiment includes a structure of holding the reaction container 100 at a predetermined position with respect to the first heating portion 12 and the second heating portion 13. Accordingly, it is possible to heat a predetermined region of the reaction container 100 by the first heating portion 12 and the second heating portion 13. More specifically, as illustrated in Figs. 3A and 3B, it is possible to heat a first region 111 by the first heating portion 12 and a second region 112 by the second heating portion 13, in a flow path 110 which configures the reaction container 100 which will be described later. In the embodiment, a structure which determines the position of the reaction container 100 is the bottom plate 17, and as illustrated in Fig. 3A, by inserting the reaction container 100 to the position at which the reaction container 100 comes into contact with the bottom plate 17, it is possible to hold the reaction container 100 at a predetermined position with respect to the first heating portion 12 and the second heating portion 13.

In a case where the reaction container 100 is mounted on the mounting portion 11, the first heating portion 12 heats the first region 111 of the reaction container 100 which will be described later to first temperature. In the example illustrated in Fig. 3A, in the main body 10, the first heating portion 12 is disposed at a position of heating the first region 111 of the reaction container 100.

The first heating portion 12 may include a mechanism which generates heat, and a member which transmits the generated heat to the reaction container 100. In the example illustrated in Fig. 2, the first heating portion 12 includes a first heater 12a and the first heat block 12b. In the embodiment, the first heater 12a is a cartridge heater, and is connected to an external power source which is not illustrated, by a conducting wire 15. The first heater 12a is inserted into the first heat block 12b, and the first heat block 12b is heated as the first heater 12a is heated. The first heat block 12b is a member which transmits the heat generated from the first heater 12a to the reaction container 100. In the embodiment, the first heat block 12b is an aluminum block.

Since the temperature of the cartridge heater is easily controlled, by using the first heater 12a as the cartridge heater, it is possible to easily stabilize the temperature of the first heating portion 12. Therefore, it is possible to more accurately realize a heat cycle. Since aluminum has high thermal conductivity, by using the first heat block 12b made of aluminum, it is possible to more efficiently heat the reaction container 100. In addition, since heating unevenness is unlikely to occur in the first heat block 12b, it is possible to realize a heat cycle having high accuracy. In addition, since the processing is easy, it is possible to mold the first heat block 12b with high accuracy, and to enhance the accuracy of heating. Therefore, it is possible to realize more accurate heat cycle.

In a case where the reaction container 100 is mounted on the mounting portion 11, it is preferable that the first heating portion 12 comes into contact with the reaction container 100. Accordingly, in a case of heating the reaction container 100 by the first heating portion 12, since it is possible to stably transmit the heat of the first heating portion 12 to the reaction container 100, it is possible to stabilize the temperature of the reaction container 100. As described in the embodiment, in a case where the mounting portion 11 is formed as a part of the first heating portion 12, it is preferable that the mounting portion 11 comes into contact with the reaction container 100. Accordingly, since it is possible to stably transmit the heat of the first heating portion 12 to the reaction container 100, it is possible to efficiently heat the reaction container 100.

In a case where the reaction container 100 is mounted on the mounting portion 11, the second heating portion 13 heats the second region 112 of the reaction container 100 to the second temperature different from the first temperature. In the example illustrated in Fig. 3A, in the main body 10, the second heating portion 13 is disposed at a position of heating the second region 112 of the reaction container 100. As illustrated in Fig. 2, the second heating portion 13 includes a second heater 13a and a second heat block 13b. The second heating portion 13 is similar to the first heating portion 12 except that the region to be heated of the reaction container 100 and the heating temperature are different from those of the first heating portion 12.

In the embodiment, the temperature of the first heating portion 12 and the second heating portion 13 is controlled by a temperature sensor and a control portion to be described later which are not illustrated. It is preferable that the temperature of the first heating portion 12 and the second heating portion 13 is set so that the reaction container 100 is heated to the predetermined temperature. In the embodiment, by controlling the first heating portion 12 to the first temperature and the second heating portion 13 to the second temperature, it is possible to heat the first region 111 of the reaction container 100 to the first temperature and the second region 112 to the second temperature. In the embodiment, the temperature sensor is a thermocouple.

The driving mechanism 20 is a mechanism which drives the mounting portion 11, the first heating portion 12, and the second heating portion 13. In the embodiment, the driving mechanism 20 includes a motor and a driving shaft which are not illustrated, and the driving shaft and the flange 16 of the main body 10 are connected to each other. The driving shaft in the embodiment is provided perpendicularly to the longitudinal direction of the mounting portion 11, and when the motor is operated, the main body 10 is rotated around the driving shaft.

The nucleic acid amplification reaction device 1 of the embodiment includes the control portion which is not illustrated. The control portion controls at least one of the first temperature, the second temperature, first time period, second time period, and the cycle number of heat cycles, which will be described later. In a case where the control portion controls the first time period and the second time period, as the control portion controls the operation of the driving mechanism 20, the time period during which the mounting portion 11, the first heating portion 12, and the second heating portion 13 are held at the predetermined position is controlled. The control portion may be provided with mechanisms which are different for each item to be controlled, or may integrally control all of the items.

The control portion in the nucleic acid amplification reaction device 1 of the embodiment is an electronic control portion, and controls all of the above-described items. The control portion of the embodiment includes a processor, such as a CPU, and a storage device, such as a read only memory (ROM) or a random access memory (RAM) which is not illustrated. Various types of programs and data for controlling each of the above-described items are stored in the storage device. In addition, the storage device has a work area in which data which is being processed in various types of processing, or a processing result, is temporarily stored.

As illustrated in the examples of Figs. 2 and 3A, in the main body 10 of the embodiment, the spacer 14 is provided between the first heating portion 12 and the second heating portion 13. The spacer 14 of the embodiment is a member which holds the first heating portion 12 or the second heating portion 13. By providing the spacer 14, the distance between the first heating portion 12 and the second heating portion 13 can be more accurately determined. In other words, the positions of the first heating portion 12 and the second heating portion 13 with respect to the first region 111 and the second region 112 of the reaction container 100 which will be described later, can be more accurately determined.

A material of the spacer 14 can be appropriately selected as necessary, but an insulating material is preferable. Accordingly, since it is possible to reduce the influence of the heat of the first heating portion 12 and the second heating portion 13 on each other, the temperature control of the first heating portion 12 and the second heating portion 13 becomes easy. In a case where the spacer 14 is an insulating material, and in a case where the reaction container 100 is mounted on the mounting portion 11, it is preferable that the spacer 14 is disposed to surround the reaction container 100 in a region between the first heating portion 12 and the second heating portion 13. Accordingly, since it is possible to suppress heat radiation from between the first heating portion 12 and the second heating portion 13 of the reaction container 100, the temperature of the reaction container 100 is further stabilized. In the embodiment, the spacer 14 is an insulating material, and in the example of Fig. 3A, the mounting portion 11 penetrates the spacer 14. Accordingly, in a case where the reaction container 100 is heated by the first heating portion 12 and the second heating portion 13, since the heat of the reaction container 100 is unlikely to be released, it is possible to further stabilize the temperature of the first region 111 and the second region 112.

The main body 10 of the embodiment includes the fixing plate 19. The fixing plate 19 is a member which holds the mounting portion 11, the first heating portion 12, and the second heating portion 13. In the example illustrated in Figs . 1B and 2, two fixing plates 19 are fitted to the flange 16, and the first heating portion 12, the second heating portion 13, and the bottom plate 17 are fixed. Since the structure of the main body 10 becomes stronger by the fixing plate 19, the main body 10 becomes unlikely to be damaged.

The nucleic acid amplification reaction device 1 of the embodiment includes the lid 50. In the examples of Figs. 1A and 3A, the mounting portion 11 is covered with the lid 50. As the mounting portion 11 is covered with the lid 50, in a case where the heating is performed by the first heating portion 12, since it is possible to suppress heat radiation from the main body 10 to the outside, it is possible to stabilize the temperature of the inside of the main body 10. The lid 50 may be fixed to the main body 10 by a fixing portion 51. In the embodiment, the fixing portion 51 is a magnet. In the examples illustrated in Figs. 1B and 2, the magnet is provided on a surface of the main body 10 which comes into contact with the lid 50. Although not illustrated in Figs. 1B and 2, a magnet is also provided in the lid 50 at a position which comes into contact with the magnet of the main body 10, and when covering the mounting portion 11 with the lid 50, the lid 50 is fixed to the main body 10 by the magnets. Accordingly, in a case where the main body 10 is driven by the driving mechanism 20, it is possible to prevent the lid 50 from being detached or moved. Therefore, since it is possible to prevent the temperature of the inside of the nucleic acid amplification reaction device 1 from being changed as the lid 50 is detached, it is possible to more accurately execute the heat cycle by reaction liquid 140 which will be described later.

It is preferable that the main body 10 has a structure having high airtightness. When the main body 10 has a structure having high airtightness, since the air of the inside of the main body 10 is unlikely to be released to the outside of the main body 10, the temperature of the inside of the main body 10 is further stabilized. In the embodiment, as illustrated in Fig. 2, a space of the inside of the main body 10 is tightly closed by two flanges 16, the bottom plate 17, two fixing plates 19, and the lid 50.

It is preferable that the fixing plate 19, the bottom plate 17, the lid 50, and the flange 16 are formed of an insulating material. Accordingly, since it is possible to further suppress the heat radiation from the main body 10 to the outside, it is possible to further stabilize the temperature of the inside of the main body 10.

### 2. Nucleic Acid Amplification Reaction Container

The nucleic acid amplification reaction container according to the embodiment includes a flow path having the longitudinal direction. The flow path is formed between a first inner wall surface and a second inner wall surface which opposes the first inner wall surface. In addition, the flow path has the longitudinal direction in a case of being seen from the direction perpendicular to the first inner wall surface.

The nucleic acid amplification reaction container according to the embodiment has a section in which the distance by which nucleic acid amplification reaction liquid comes into contact with both of the first inner wall surface and the second inner wall surface that form the flow path in a case where the nucleic acid amplification reaction liquid is guided therein, that is, the length in the direction parallel to first inner wall surface in the section of the flow path orthogonal to the longitudinal direction, is 5 times or more the distance between the first inner wall surface and the second inner wall surface. In addition, in the nucleic acid amplification reaction container according to the embodiment, the nucleic acid amplification reaction liquid and gas can be disposed in the flow path.

Hereinafter, as an example of the nucleic acid amplification reaction container according to the embodiment, several aspects will be described using the reaction container 100, a reaction container 101, a reaction container 102, and a reaction container 103.

### 2.1. First Aspect

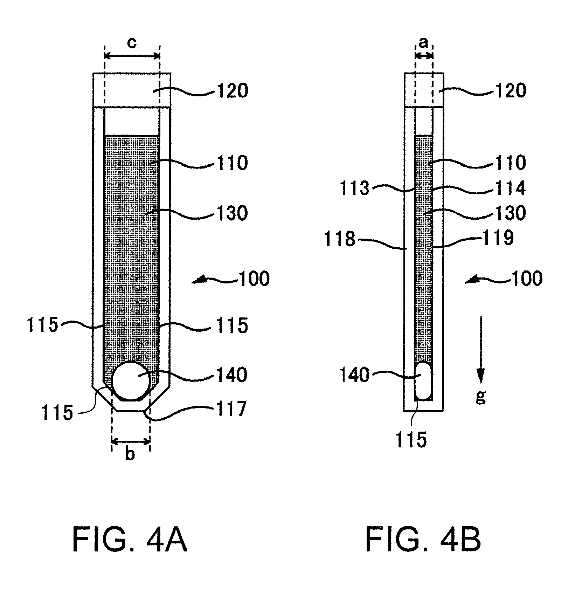

Figs. 4A and 4B are schematic views of a section of the reaction container 100 according to the aspect. Fig. 4A is a schematic view of a section cut by a surface parallel to a first inner wall surface 113 and a second inner wall surface 114 which oppose each other in the reaction container 100 along the longitudinal direction, and Fig. 4B is a section cut by a surface orthogonal to the first inner wall surface 113 and the second inner wall surface 114 of the reaction container 100 along the longitudinal direction.

As illustrated in Figs. 4A and 4B, the reaction container 100 according to the first aspect includes the flow path 110 and a lid body 120. The flow path 110 is divided by the first inner wall surface 113, the second inner wall surface 114 which opposes the first inner wall surface 113, and a third inner wall surface 115 which links the first inner wall surface 113 and the second inner wall surface 114. In addition, the longitudinal direction of the flow path 110 is the direction along the first inner wall surface 113 (and the second inner wall surface 114), and as illustrated in Fig. 4A, in a case of being seen from the direction orthogonal to the first inner wall surface 113, the longitudinal direction is the direction which extends to be longer than the width c of the flow path 110, and the direction which links one open end and the other closed end (bottom portion 117 side).

In addition, the third inner wall surface 115 which divides the flow path 110 includes one pair of parallel wall surfaces which extend in the longitudinal direction, and a bottom surface formed by the bottom portion 117. In addition, one open end side of the flow path 110 is divided by a surface of the lid body 120 as the lid body 120 is mounted and sealed.

At least the nucleic acid amplification reaction liquid (hereinafter, simply referred to as "reaction liquid 140") and gas 130 are guided into the flow path 110, and the flow path 110 is sealed with the lid body 120. In addition, in the flow path 110, liquid (not illustrated) in which a specific gravity is smaller than that of the reaction liquid 140 and which is not mingled with the reaction liquid 140, may be guided therein. However, even in this case, the amount of each type of liquid is appropriately adjusted so that the gas 130 is present in the flow path 110.

The shape of the reaction container 100 is flat, and has the longitudinal direction (the vertical direction in Figs. 4A and 4B, and the direction parallel to the first inner wall surface 113 and the second inner wall surface 114). The longitudinal direction of the flow path 110 is the direction in which the reaction liquid 140 (nucleic acid amplification reaction liquid) moves while the nucleic acid amplification reaction device is operated.

The reaction container 100 is sealed with the lid body 120. In the reaction container 100, the center of the bottom portion 117 of the reaction container 100 has a shape which protrudes toward the outside so that a liquid droplet of the reaction liquid 140 input into the container is likely to be positioned at the center of the bottom portion in the bottom portion 117 of the container.

The first inner wall surface 113 and the second inner wall surface 114 are planes which oppose each other. The distance (width a illustrated in Fig. 4B) between the first inner wall surface 113 and the second inner wall surface 114 is the distance by which one liquid droplet of the reaction liquid 140 poured into the reaction container 100 comes into contact with both of the first inner wall surface 113 and the second inner wall surface 114 at the same time. In addition, in a state where the reaction liquid 140 comes into contact with both of the first inner wall surface 113 and the second inner wall surface 114 at the same time, the reaction liquid 140 may be in contact with or may not be in contact with the third inner wall surface 115.

The shape of the section of the flow path 110 cut by a surface orthogonal to the longitudinal direction of the flow path 110 will be described. In the reaction container 100 of the aspect, the section of the flow path 110 cut by the surface orthogonal to the longitudinal direction has a shape of substantially rectangular parallelepiped at a part other than the bottom portion 117. In addition, the length in the direction parallel to the first inner wall surface 113 (second inner wall surface 114) of the section is 5 times or more the distance between the first inner wall surface 113 and the second inner wall surface 114. In other words, the width c in Fig. 4A is 5 times or more the width a in Fig. 4B.

The shape of the section can be regulated by an aspect ratio (flat ratio). In the specification, the aspect ratio is a value obtained by dividing the size in the direction (lateral direction) (short axis) orthogonal to the longitudinal direction by the size in the longitudinal direction (long axis), in the shape having the longitudinal direction. In this case, it can be said that the aspect ratio (c/a) of the section of the flow path 110 cut by the surface orthogonal to the longitudinal direction of the above-described flow path 110 is equal to or greater than 5. In addition, in the example, the width c is the distance between the third inner wall surfaces 115 which link the first inner wall surface 113 and the second inner wall surface 114, in the example of Fig. 4A.

The length in the direction parallel to the first inner wall surface 113 of the section of the flow path 110 cut by the surface orthogonal to the longitudinal direction is preferably 6 times or more the distance between the first inner wall surface 113 and the second inner wall surface 114, more preferably 7 times or more, and still more preferably 8 times or more. In other words, the aspect ratio of the section of the flow path 110 cut by the surface orthogonal to the longitudinal direction of the flow path 110 is preferably 6 times or more, more preferably 7 times or more, and still more preferably 8 times or more.

As the flow path 110 has such a flat shape, a smaller amount of the reaction liquid 140 can come into contact with both of the first inner wall surface 113 and the second inner wall surface 114 at the same time.

The volume of the reaction liquid 140 and the gas 130 guided into the flow path 110 is not particularly limited as long as the volume of the gas 130 takes 50% or more of the capacity of the flow path 110. According to this, it is possible to dispose the reaction liquid 140 in any one of two different temperature regions. The volume of the guided reaction liquid 140 is 50% or less of the capacity of the flow path 110, preferably 45% or less, more preferably 40% or less, and still more preferably 35% or less. In addition, although the volume depends on the distance (width a illustrated in Fig. 4B) between the first inner wall surface 113 and the second inner wall surface 114, the volume is sufficient if the volume which comes into contact with both of the first inner wall surface 113 and the second inner wall surface 114 at the same time is guided in, and for example, the volume of the guided reaction liquid 140 is 5% or more of the capacity of the flow path 110, preferably 10% or more, more preferably 15% or more, and still more preferably 20% or more.

Meanwhile, the distance (width a) between the first inner wall surface 113 and the second inner wall surface 114 is from 0.2 mm to 3 mm, preferably from 0.5 mm to 2.5 mm, and more preferably from 0.5 mm to 1 mm.

A state where the reaction liquid 140 is in contact with both of the first inner wall surface 113 and the second inner wall surface 114 at the same time, can be obtained by appropriately adjusting the volume of the reaction liquid 140 and the distance between the first inner wall surface 113 and the second inner wall surface 114. In addition, a state where the gas 130 is present 50% or more in the flow path 110, can be designed, for example, considering the actual size of the reaction container 100, in a case where only the reaction liquid 140 and the gas 130 are guided into the flow path 110 (that is, the oil or the like is not guided therein, and the volume of the reaction liquid 140 can be at a maximum of 50%). In this manner, the size of the reaction container 100 and the shape of the flow path 110 of the aspect, can be appropriately designed considering the volume of the guided reaction liquid 140.

The volume of the reaction liquid 140 guided into the flow path 110 is not particularly limited as long as the shape of the flow path 110 is designed to satisfy the above-described conditions. The volume of the reaction liquid 140 guided into the flow path 110 is, for example, from 1 µl to 100 µl, preferably from 5 µl to 50 µl, more preferably from 10 µl to 40 µl, and still more preferably from 20 µl to 30 µl. When the volume is in this range, the nucleic acid in the reaction liquid 140 is easily amplified.

The shape of the flow path 110 in a case where the reaction liquid 140 having the volume of the above-described range is guided in will be described using several examples. A case where the shape of the flow path 110 is a shape of a flat rectangular parallelepiped having the longitudinal direction, the thickness (corresponds to the above-described distance between the first inner wall surface 113 and the second inner wall surface 114) is D, the length in the longitudinal direction is L, and the length (corresponds to the above-described width c) in the direction orthogonal to the longitudinal direction is W, is considered (in this case, L > D).

In a case where the amount guided of the reaction liquid 140 is 10 µl, for example, when D = 0.2 mm and W = 5 mm, and when L is equal to or greater than 2 cm, it is possible to satisfy the above-described condition. In this case, the above-described aspect ratio becomes W/D = 25. Similarly, in a case where the amount guided of the reaction liquid 140 is 50 µl, for example, when D = 1 mm and W = 5 mm, and when L is equal to or greater than 2 cm, it is possible to satisfy the above-described condition. In this case, the above-described aspect ratio becomes W/D = 5. In a case where the amount guided of the reaction liquid 140 is 100 µl, for example, when D = 1 mm and W = 1 cm, and when L is equal to or greater than 2 cm, it is possible to satisfy the above-described condition. In this case, the above-described aspect ratio becomes W/D = 10. In addition, the amount guided of the reaction liquid 140 may be decided by the shape (D, W, L) of the container.

In addition, in the aspect, the width c is not particularly limited. In other words, in the reaction container 100 of the aspect, since the gas (air or the like) of which the volume is 50% or more is guided into the flow path 110, even if one liquid droplet of the reaction liquid 140 comes into contact with the third inner wall surfaces 115 at the same time, it is possible to extremely easily perform the movement in the flow path 110 of the reaction liquid 140 by the action of the gravity. It can be considered that this is because, as the above-described aspect ratio of the section is equal to or greater than 5, a meniscus of the reaction liquid 140 is unlikely to be formed, and the surface (interface between the reaction liquid 140 and the gas 130) of the reaction liquid 140 is likely to be broken.

If the reaction container 100 of the aspect is used, when the reaction liquid 140 moves in the longitudinal direction of the reaction container 100, the reaction liquid 140 is always in contact with both of the opposing inner walls of the reaction container 100. Furthermore, since the shape of the flow path 110 is flat, the contact state between the reaction liquid 140, the first inner wall surface 113, and the second inner wall surface 114 becomes surface contact. In addition, in the section of the flow path 110 orthogonal to the longitudinal direction, the section in which the length in the direction parallel to the first inner wall surface 113 is 5 times or more the distance between the first inner wall surface 113 and the second inner wall surface 114, is provided. In other words, the reaction liquid 140 is nipped by the first inner wall surface 113 and the second inner wall surface 114 of the reaction container 100, and the liquid droplet of the reaction liquid 140 is compressed in the thickness direction, and the size thereof expands in the direction orthogonal to the thickness direction.

According to this, the contact area can increase. Therefore, since the heat from the heating portion can be directly transmitted to the reaction liquid 140 in the wide area from the wall surface of the reaction container 100, heat transmission efficiency is high. Accordingly, direct heat exchange between the reaction liquid 140 and the inner wall surface can be excellently performed, and the reaction liquid 140 can achieve the predetermined temperature extremely fast. Accordingly, amplification of the nucleic acid can be performed at high speed.

In addition, a first wall 118 and a second wall 119 which form the first inner wall surface 113 and the second inner wall surface 114 easily perform the heat exchange inside and outside the container, as the thickness becomes thinner. In other words, the thickness of at least one of the first wall 118 and the second wall 119 is preferably from 0.01 mm to 0.5 mm, more preferably from 0.05 mm to 0.4 mm, and still more preferably from 0.1 mm to 0.3 mm.

In the reaction container 100 according to the aspect, since a drop position of the reaction liquid 140 is stable in the width direction of the reaction container 100, it is easier to perform fluorescence measurement of the reaction liquid 140 from the end portion (bottom portion 117 in the example illustrated in the drawing) of the reaction container 100 in the longitudinal direction. In addition, since the drop position of the reaction liquid 140 is stable in the thickness direction of the reaction container 100, as illustrated in the following modification example 1, the reaction container 100 according to the aspect is similar even when performing the fluorescence measurement of the reaction liquid 140 from the horizontal direction.

With reference to Figs. 3A and 3B again, the first region 111 of the reaction container 100 is a region of a part of the flow path 110, and is heated to the first temperature by the first heating portion 12. The second region 112 is a region of a part of the flow path 110 different from the first region 111, and is heated to the second temperature by the second heating portion 13. In the reaction container 100 of the aspect, the first region 111 is a region including one end portion of the flow path 110 in the longitudinal direction, and the second region 112 is a region including the other end portion of the flow path 110 in the longitudinal direction. In the example illustrated in Figs. 3A and 3B, the region surrounded by a dotted line including the end portion on the lid body 120 side of the flow path 110 is the second region 112, and a region surrounded by a dotted line including the end portion on a side far from the lid body 120 is the first region 111.

The flow path 110 is filled with the gas 130 and the reaction liquid 140. Since the gas 130 is, for example, the atmosphere (air) and is hydrophobic, as illustrated in Figs. 4A and 4B, the reaction liquid 140 is held in a state of the liquid droplet (liquid storing) in the gas 130. Since the specific gravity of the reaction liquid 140 is greater than that of the gas 130, the reaction liquid 140 is positioned in a region of the lowest portion in the gravity direction of the flow path 110.

The reaction liquid 140 is liquid including components which are necessary for the reaction. In a case where the reaction is PCR, a DNA (target nucleic acid) amplified by the PCR, a DNA polymerase which is necessary for amplifying the DNA, and a primer are included. In addition, in the reaction liquid 140, the fluorescent probe or the like for monitoring the progress of the PCR may be included.

The material of the reaction container 100 is not particularly limited as long as deformation or the like does not occur due to the temperature of the PCR reaction, and examples thereof can include polyester, polypropylene, polyvinyl alcohol, polyvinyl acetate, polycarbonate, acrylic resin, and a high molecule, such as a copolymer of these materials. The materials of the container and the lid body 120 may be different from each other. When selecting a thermoplastic resin, such as polypropylene, as the material of the reaction container 100, the manufacturing can become easy by injection molding or the like.

Furthermore, at least one of the walls which form the flow path 110 including the first wall 118 and the second wall 119, is formed of a transparent material, and may have light transmission properties. In this manner, in a case where the reaction container 100 is used in the heat cycle processing, for example, the fluorescence of the fluorescent probe can be easily measured. When at least one of the first wall 118 and the second wall 119 is transparent, since a state where the reaction liquid 140 moves can be observed from the outside, it is possible to visually confirm whether or not the heat cycle processing is appropriately performed. Here, the degree of "transparency" may be a degree by which the reaction liquid 140 can be visually confirmed in a case where the members are employed in the nucleic acid amplification reaction device 1, and the heat cycle processing is performed.

### 2.2. Second Aspect

Figs. 5A to 5C are schematic views of the section of the reaction container 101 according to the second aspect. Fig. 5A is an upper view when the reaction container 101 is viewed from the longitudinal direction, and is a schematic view of the section cut by the surface parallel to the first inner wall surface 113 and the second inner wall surface 114 which oppose each other in the reaction container 101, and Fig. 5B is an upper view when the reaction container 101 is viewed from the longitudinal direction, and is a schematic view of the section cut by the surface orthogonal to the first inner wall surface 113 and the second inner wall surface 114 of the reaction container 101. In Figs. 5A and 5B, dashed lines illustrating the corresponding sections are drawn in each of the upper views, and the lid body 120 is omitted. Fig. 5C is a schematic view of the section cut by the surface orthogonal to the first inner wall surface 113 and the second inner wall surface 114 which oppose each other in the reaction container 101.

The reaction container 101 of the aspect is similar to the above-described reaction container 100 of the first aspect except that the shape of the lid body 120 is different and the center of the bottom portion does not protrude toward the outside unlike the reaction container 100. Therefore, similar members and parts will be given the same reference numerals, and the description thereof will be omitted.

The reaction container 101 of the second aspect includes the lid body 120 which seals the flow path 110, and the lid body 120 has a shape including the cylindrical surface which considers the longitudinal direction as a shaft. In other words, the flow path 110 is formed in a flat shape, but the lid body 120 is formed to include a columnar shape or a cylindrical shape. In the example of Fig. 5C, the lid body 120 has a shape of a columnar plug which seals one end side of the flow path 110. The lid body 120 may have a shape of a plug which is illustrated, or may have a shape of a cap. Furthermore, the lid body 120 may be provided with a screw mechanism or the like.

The reaction container 101 forms the flat flow path 110, and the lid body 120 has a shape including the cylindrical surface which considers the longitudinal direction as a shaft. Therefore, compared to the reaction container 100, the reaction container 101 is easily handled by the user, and for example, workability can be improved.

### 2.3. Third Aspect

Figs. 6A to 6C are schematic views of the section of the reaction container 102 according to the third aspect. Figs. 6A and 6B are upper views when the reaction container 101 is viewed from the longitudinal direction, and are schematic views of the section cut by the surface orthogonal to the first inner wall surface 113 and the second inner wall surface 114 which oppose each other in the reaction container 101. In Figs. 6A and 6B, dashed lines illustrating the position of the corresponding section are drawn in each of the upper views. Fig. 6C is a schematic view of the section cut by the surface parallel to the first inner wall surface 113 and the second inner wall surface 114 which oppose each other in the reaction container 101. In Fig. 6C, a film portion 124 for forming the first inner wall surface 113 and the second inner wall surface 114 is virtually drawn. In addition, in Figs. 6A to 6C, the lid body 120 is omitted.

The reaction container 102 of the third aspect is similar to the above-described reaction container 101 of the second aspect except that the structure of the first wall 118 and the second wall 119 is different. Therefore, similar members and parts will be given the same reference numerals, and the description thereof will be omitted.

In the above-described reaction container 100 and the reaction container 101, the first wall 118 and the second wall 119 are molded to be integrated with other parts. However, the flow path 110 of the reaction container 102 of the third aspect is formed to include an edge portion 122 and the film portion 124. As illustrated in Fig. 6A, the edge portion 122 forms the third inner wall surface 115 which regulates the flow path 110. In addition, as illustrated in Fig. 6A, the film portion 124 forms at least a part of the first inner wall surface 113 and the second inner wall surface 114 which regulate the flow path 110. A part of the first inner wall surface 113 and the second inner wall surface 114 may be formed of the edge portion 122.

In the description of the above-described reaction container 100, it is described that the thickness of at least one of the first wall 118 and the second wall 119 is preferably from 0.01 mm to 0.5 mm. When the thickness of the first wall 118 and the second wall 119 is thinner to the extent of 0.1 mm, for example, there is a case where it becomes difficult to integrally perform the forming by the injection molding as described in the example illustrated in Figs. 5A to 5C. However, similar to the reaction container 102 of the aspect, by forming the edge portion 122 to have the thickness by which the injection molding is possible, and by forming the first wall 118 and the second wall 119 as separated bodies by the molded film portion 124, the reaction container 102 having extremely thin first wall 118 and the second wall 119 can be obtained.

The thickness of the film portion 124 is not particularly limited, but, for example, from 0.01 mm to 0.5 mm, preferably from 0.02 mm to 0.3 mm, more preferably from 0.03 mm to 0.2 mm, and still more preferably from 0.04 mm to 0.1 mm.

The film portion 124 may have any of a single layer structure and a multi-layer structure. The multi-layer structure is more preferable since various functions can be realized by each layer. Examples of such functions include gas barrier properties, adhesiveness, welding properties, strength (rigidity), and visually recognizing properties. The material of the film portion 124 is not particularly limited, and for example, polyester, polypropylene, polyvinyl alcohol, polyvinyl acetate, polycarbonate, acrylic resin, and a high molecule, such as a copolymer of these materials, can be appropriately included in accordance with predetermined functions.

The adhesion of the edge portion 122 and the film portion 124 may be performed using the adhesive, and when the material of the adhesion surface between the edge portion 122 and the film portion 124 is the same type of the thermoplastic resin, it is possible to perform the welding by heat welding, and thus, there is a case where the manufacturing becomes easier.

In the reaction container 102 of the third aspect, the first wall 118 and the second wall 119 which form the first inner wall surface 113 and the second inner wall surface 114 are formed to be extremely thin by the film portion 124. Therefore, the heat exchange inside and outside the container via the first wall 118 and the second wall 119 is easily performed. Therefore, it is possible to reduce the heat transmission distance, and to decrease the difference in temperature between the outer side and the inner side. Therefore, for example, the nucleic acid amplification reaction liquid can achieve the set temperature of the external heater faster.

### 2.4. Fourth Aspect

Fig. 7 is a perspective view schematically illustrating the reaction container 103 according to the fourth aspect. In Fig. 7, the film portion 124 and the lid body 120 are omitted. Figs. 8A and 8B are schematic views of the section of the reaction container 103. Figs. 8A and 8B are respectively a section taken along line A-A and a section taken along line B-B of Fig. 7.

In the above-described reaction container 103 of the third aspect, the direction in which the lid body 120 which is not illustrated is inserted is along the longitudinal direction of the flow path 110, but in the reaction container 103 of the fourth aspect, the direction in which the lid body 120 which is not illustrated is inserted is along the direction perpendicular to the longitudinal direction of the flow path 110. The reaction container 103 of the fourth aspect is similar to the reaction container 103 of the third aspect except that the shape of the edge portion 122 varies as the related point varies. Therefore, similar members and parts will be given the same reference numerals, and the description thereof will be omitted.

In the reaction container 103, a communication path 126 is formed on one end side of the flow path 110. The communication path 126 forms a path for guiding the reaction liquid 140 into the flow path 110. In the example illustrated in the drawing, the communication path 126 communicates with an opening 128 on which the lid body 120 (not illustrated) is mounted. The film portion 124 is disposed on an upper surface 122a and a lower surface 122b of the edge portion 122, as illustrated in Fig. 8. Therefore, the first wall 118 and the second wall 119 which respectively form the first inner wall surface 113 and the second inner wall surface 114 of the flow path 110, are formed by the film portion 124, and the third inner wall surface 115 is formed by the edge portion 122. In addition, the communication path 126 is formed by the edge portion 122 and the film portion 124.

As illustrated in the drawing, the communication path 126 is formed by using a notch formed on the lower surface 122b side of the edge portion 122. The depth of the notch is, for example, approximately half of the thickness (distance between the first inner wall surface 113 and the second inner wall surface 114) of the edge portion 122, and for example, in a case where the thickness of the edge portion 122 is 0.5 mm, the depth can be approximately 0.25 mm.

As the flow cross section area of the communication path 126 decreases, the reaction liquid 140 is unlikely to infiltrate into the communication path 126 by the action of the gravity. Therefore, the flow path 110 in the reaction container 103 does not include the communication path 126. However, in a case where the flow cross section area of the communication path 126 increases and the reaction liquid 140 can infiltrate into the communication path 126 by the action of the gravity, the communication path 126 may also be the flow path 110. Furthermore, when the tip end of the lid body 120 which is not illustrated is designed to be fixed at the position of the upper surface 122a of the edge portion 122, it is also possible to use the space regulated by the film portion 124 and the lid body 120 on the lower surface 122b side, as the flow path 110. In addition, according to the lid body 120, the position opposite to the flow path 110 of the communication path 126 may be sealed.

The reaction container 103 is stabilized in a state where the reaction container 103 is placed on a plane, such as a desk, in addition to the characteristics of each of the above-described reaction containers. Therefore, for example, work of guiding the reaction liquid 140 into the flow path 110 can become easy.

### 3. Heat Cycle Processing in which Nucleic Acid Amplification Reaction Device is Used

The reaction method for amplifying nucleic acid of the embodiment includes: guiding the reaction liquid 140 into the above-described reaction container (for example, reaction container 100), and disposing at least the reaction liquid 140 and the gas 130 into the flow path 110; heating the first region 111 of the reaction container 100 to first temperature; heating the second region 112 of the reaction container 100 to the second temperature different from the first temperature; moving the reaction liquid 140 in the flow path 110, and moving the reaction liquid 140 from the first region 111 to the second region 112; and moving the reaction liquid 140 in the flow path 110, and moving the reaction liquid 140 from the second region 112 to the first region 111. According to the reaction method for amplifying nucleic acid, the nucleic acid can be amplified at high speed. Hereinafter, the reaction method for amplifying nucleic acid will be described in more detail.

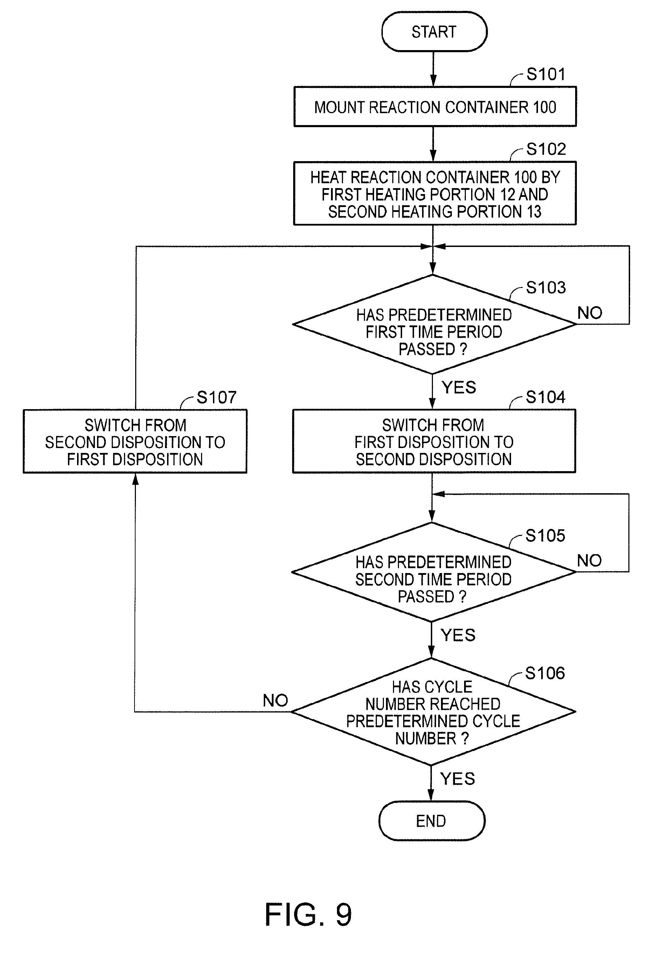

In Figs. 3A and 3B used in the description above, Fig. 3A illustrates a first disposition, and Fig. 3B illustrates a second disposition. In addition, Fig. 9 is a flowchart illustrating an order of the heat cycle processing in which the nucleic acid amplification reaction device 1 is used in the embodiment. Hereinafter, in a case where the reaction container 100 according to the first aspect of the above-described embodiment is used, the heat cycle processing in which the nucleic acid amplification reaction device 1 according to the embodiment will be described.

Hereinafter, with reference to Figs. 3A, 3B, and 9, the heat cycle processing in which the nucleic acid amplification reaction device 1 according to the embodiment is used will be described. In Figs. 3A and 3B, the direction of the arrow g (downward direction in the drawing) is the direction in which the gravity acts. In the embodiment, a case where a shuttle PCR (two-stepped temperature PCR) is performed will be described as an example of the heat cycle processing. In addition, each process to be described below illustrates an example of the heat cycle processing. As necessary, the order of processes may be exchanged, two or more processes may be performed sequentially or in parallel, or a process may be added.

The shuttle PCR is a technique of amplifying the nucleic acid in the reaction liquid by repeating two-stepped temperature processing of the high temperature and the low temperature with respect to the reaction liquid. A double-stranded DNA is dissociated in the processing of high temperature, and annealing (reaction in which a primer is bonded to a single-stranded DNA) and extension reaction (reaction in which a complemental strand of DNA is formed using the primer as a start) are performed in the processing of low temperature.

In general, in the shuttle PCR, the high temperature is temperature from 80°C to 100°C, and the low temperature is temperature from 50°C to 70°C. At each temperature, the processing is performed for a predetermined time period, and the holding time period at high temperature is generally shorter than the holding time period at low temperature. For example, high temperature may be held approximately from 1 seconds to 10 seconds, low temperature may be held approximately from 10 seconds to 60 seconds, and the time period may be longer than this according to the condition of the reaction.

In addition, since appropriate time period, temperature, and cycle number (the number of repeating the high temperature and the low temperature) vary according to the type or the amount of a reagent to be used, it is preferable to perform the reaction after deciding the appropriate protocol considering the type of the reagent or the amount of the reaction liquid 140.

First, the reaction container 100 according to the embodiment is mounted on the mounting portion 11 (step S101). In the embodiment, after guiding the reaction liquid 140 into the flow path 110 filled with the gas 130, the reaction container 100 sealed with the lid body 120 is mounted on the mounting portion 11. The reaction liquid 140 can be guided in using a micropipette or ink jet type dispensing device. In a state where the reaction container 100 is mounted on the mounting portion 11, the first heating portion 12 is in contact with the reaction container 100 and the second heating portion 13 is in contact with the reaction container 100 respectively at a position which includes the first region 111 and at a position which includes the second region 112. In the embodiment, as illustrated in Fig. 3A, as the reaction container 100 is mounted to be in contact with the bottom plate 17, the reaction container 100 can be held at a predetermined position with respect to the first heating portion 12 and the second heating portion 13.

In the embodiment, in step S101, the disposition of the mounting portion 11, the first heating portion 12, and the second heating portion 13 is the first disposition. As illustrated in Fig. 3A, the first disposition is disposition in which the first region 111 is below the second region 112 in the vertical direction, and in the embodiment, the first disposition is disposition in which the first region 111 of the reaction container 100 is positioned in the lowest portion of the flow path 110 in the direction in which the gravity acts. Therefore, in a case where the mounting portion 11, the first heating portion 12, and the second heating portion 13 are disposed in the predetermined disposition, the first region 111 is a region of a part of the flow path 110 which is positioned in the lowest portion of the flow path 110 in the direction in which the gravity acts. In the first disposition, since the first region 111 is positioned in the lowest portion of the flow path 110 in the direction in which the gravity acts, the reaction liquid 140 having a specific gravity higher than that of the gas 130 is positioned in the first region 111. In the embodiment, when the reaction container 100 is mounted on the mounting portion 11, the mounting portion 11 is covered with the lid 50, and the nucleic acid amplification reaction device 1 is operated. In the embodiment, when the nucleic acid amplification reaction device 1 is operated, step S102 and step S103 are started.

In step S102, the reaction container 100 is heated by the first heating portion 12 and the second heating portion 13. The first heating portion 12 and the second heating portion 13 heat the different region of the reaction container 100 to different temperature. In other words, the first heating portion 12 heats the first region 111 to the first temperature, and the second heating portion 13 heats the second region 112 to the second temperature. Accordingly, between the first region 111 and the second region 112 of the flow path 110, a temperature gradient in which the temperature gradually changes between the first temperature and the second temperature is formed. In the embodiment, the first temperature is temperature which is relatively high among the temperatures appropriate for reaction to be a target in the heat cycle processing, and the second temperature is temperature which is relatively low among the temperatures appropriate for reaction to be a target in the heat cycle processing. Therefore, in step S102 of the embodiment, the temperature gradient in which the temperature decreases from the first region 111 to the second region 112 is formed. Since the heat cycle processing of the embodiment is the shuttle PCR, it is preferable that the first temperature is the temperature appropriate for dissociation of double-stranded DNA, and the second temperature is the temperature appropriate for annealing and extension reaction.

In step S102, since the disposition in which the mounting portion 11, the first heating portion 12, and the second heating portion 13 is the first disposition, when heating the reaction container 100 in step S102, the reaction liquid 140 is heated to the first temperature. Therefore, in step S102, the reaction is performed at the first temperature with respect to the reaction liquid 140.

In step S103, in the first disposition, it is determined whether or not the first time period has passed. In the embodiment, the determination is performed by the control portion which is not illustrated. The first time period is the time period during which the mounting portion 11, the first heating portion 12, and the second heating portion 13 are held in the first disposition. In the embodiment, in a case where step S103 is performed following the mounting in step S101, that is, in a case where step S103 is performed one time, it is determined whether or not the time period while operating the nucleic acid amplification reaction device 1 has reached the first time period. In the first disposition, since the reaction liquid 140 is heated to the first temperature, it is preferable that the first time period is the time period during which the reaction liquid 140 is reacted at the first temperature in the reaction to be a target. In the embodiment, it is preferable that the first time period is the time period necessary for dissociation of double-stranded DNA.

In step S103, in a case where it is determined that the first time period has passed (YES), the process moves to step S104. In a case where it is determined that the first time period has not passed (NO), step S103 is repeated.

In step S104, the main body 10 is driven by the driving mechanism 20, and the disposition of the mounting portion 11, the first heating portion 12, and the second heating portion 13 is switched from the first disposition to the second disposition. The second disposition is disposition in which the second region 112 is below the first region 111 in the vertical direction, and in the embodiment, the second disposition is disposition in which the second region 112 is positioned in the lowest portion of the flow path 110 in the direction in which the gravity acts. In other words, in a case where the mounting portion 11, the first heating portion 12, and the second heating portion 13 are disposed in a predetermined disposition different from the first disposition, the second region 112 is a region which is positioned in the lowest portion of the flow path 110 in the direction in which the gravity acts.

In step S104 of the embodiment, the disposition of the mounting portion 11, the first heating portion 12, and the second heating portion 13 is switched from the state of Fig. 3A to the state of Fig. 3B. In the nucleic acid amplification reaction device 1 of the embodiment, the driving mechanism 20 drives and rotates the main body 10 by the control of the control portion. When the flange 16 is driven to rotate by the motor around the driving shaft, the mounting portion 11, the first heating portion 12, and the second heating portion 13 which are fixed to the flange 16 are rotated. Since the driving shaft is a shaft in the direction perpendicular to the longitudinal direction of the mounting portion 11, when the driving shaft rotates by the operation of the motor, the mounting portion 11, the first heating portion 12, and the second heating portion 13 are rotated. In the example illustrated in Figs. 3A and 3B, the main body 10 is rotated by 180°. Accordingly, the disposition of the mounting portion 11, the first heating portion 12, and the second heating portion 13 is switched from the first disposition to the second disposition.

In step S104, since the positional relationship of the first region 111 and the second region 112 in the direction in which the gravity acts becomes reverse to the first disposition, the reaction liquid 140 moves from the first region 111 to the second region 112 by the action of gravity. In a case where the disposition of the mounting portion 11, the first heating portion 12, and the second heating portion 13 achieves the second disposition, when the control portion stops the operation of the driving mechanism 20, the disposition of the mounting portion 11, the first heating portion 12, and he second heating portion 13 is held to be the second disposition. When the disposition of the mounting portion 11, the first heating portion 12 , and the second heating portion 13 has achieved the second disposition, step S105 is started.

In step S105, in the second disposition, it is determined whether or not the second time period has passed. The second time period is the time period during which the mounting portion 11, the first heating portion 12, and the second heating portion 13 are held in the second disposition. In the embodiment, since the second region 112 is heated to the second temperature in step S102, in step S105 of the embodiment, it is determined whether or not the time period after the disposition of the mounting portion 11, the first heating portion 12, and the second heating portion 13 has achieved the second disposition has achieved the second time period. In the second disposition, since the reaction liquid 140 is held in the second region 112, during the time period during which the main body 10 is held in the second disposition, the reaction liquid 140 is heated to the second temperature. Therefore, it is preferable that the second time period is the time period of heating the reaction liquid 140 to the second temperature in the reaction to be a target. In the embodiment, it is preferable that the second time period is the time period necessary for the annealing and the extension reaction.

In step S105, in a case where it is determined that the second time period has passed (YES), the process moves to step S106. In a case where it is determined that the second time period has not passed (NO), step S105 is repeated.

In step S106, it is determined whether or not the number of heat cycles has reached the predetermined number of cycles. Specifically, it is determined whether or not the predetermined number of the orders from step S103 to step S105 has been completed. In the embodiment, the number of completions of step S103 and step S105 is determined by the number of determinations of "YES". When the process from step S103 to step S105 is performed one time, since one heat cycle is performed with respect to the reaction liquid 140, the number of performances from step S103 to step S105 can be the cycle number of the heat cycles. Therefore, by step S106, it is possible to determine whether or not the heat cycles for the reaction to be a target are performed necessary number of times.

In step S106, in a case where it is determined that the heat cycles are performed expected cycle number of times (YES), the processing is completed (END). In a case where it is determined that the heat cycles are not performed expected cycle number of times (NO), the process moves to step S107.

In step S107, the disposition of the mounting portion 11, the first heating portion 12, and the second heating portion 13 is switched from the second disposition to the first disposition. By driving the main body 10 by the driving mechanism 20, the disposition of the mounting portion 11, the first heating portion 12, and the second heating portion 13 can be the first disposition. When the disposition of the mounting portion 11, the first heating portion 12, and the second heating portion 13 achieves the first disposition, step S103 is started.

In a case where step S103 is performed following step S107, that is, in step S103 after the second performance, it is determined whether or not the time period after the disposition of the mounting portion 11, the first heating portion 12, and the second heating portion 13 achieves the first disposition, reaches the first time period.

It is preferable that the direction in which the mounting portion 11, the first heating portion 12, and the second heating portion 13 are rotated by the driving mechanism 20 is the opposite direction in the rotation in step S104 and in step S107. Accordingly, since torsion generated in the wiring, such as the conducting wire 15, due to the rotation can be eliminated, deterioration of the wiring can be suppressed. It is preferable that the direction of the rotation is reversed in each one operation by the driving mechanism 20. Accordingly, compared to a case where the rotation in the same direction is continuously performed plural times, the degree of the torsion of the wiring can be reduced.

### 4. Modification Example

Hereinafter, modification examples according to the embodiment will be described.

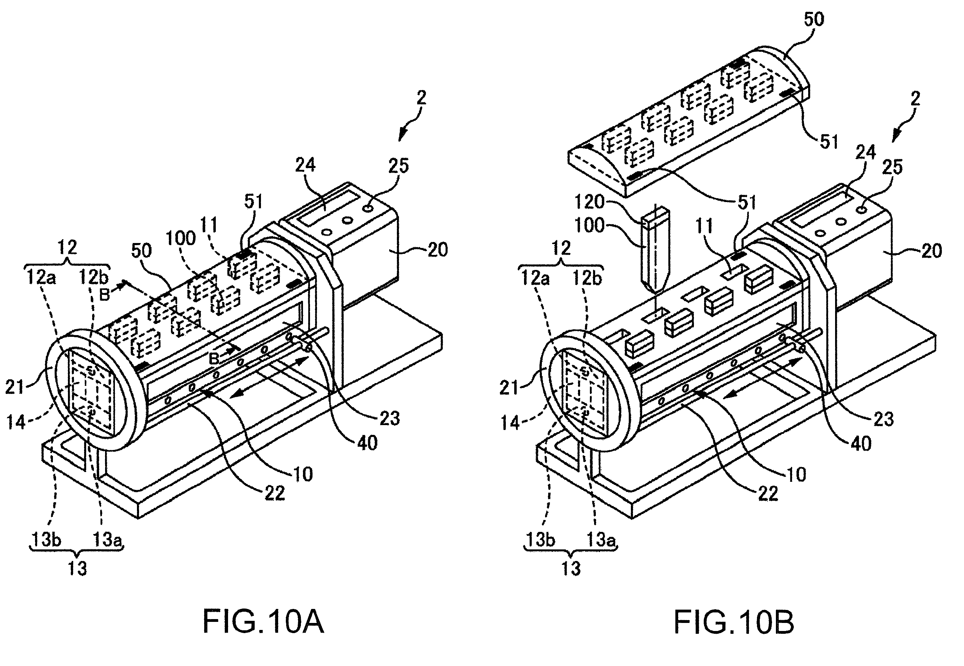

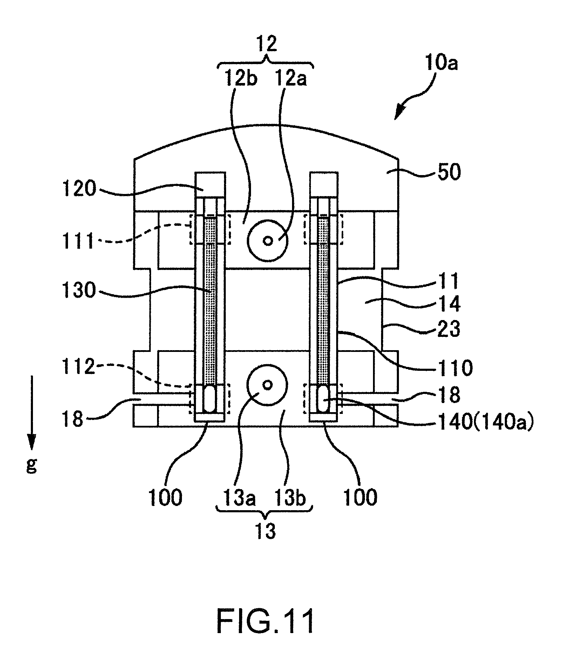

Figs. 10A and 10B are perspective views of a nucleic acid amplification reaction device 2 according to the modification example. Fig. 10A illustrates a state where the lid 50 is closed, and Fig. 10B illustrates a state where the lid 50 is open. Fig. 11 is a sectional view schematically illustrating the section taken along line B-B of Fig. 10A of a main body 10a of the nucleic acid amplification reaction device 2 according to the modification example. The following modification examples can be arbitrarily combined with each other as long as the configurations do not conflict therebetween. In the following, configurations different from those of the above-described embodiments will be described in detail, configurations similar to those of the embodiments will be given the same reference numerals, and the description thereof will be omitted. The modification examples are described focusing on a case where the above-described reaction container 100 is used, but the reaction container 101, the reaction container 102, and the reaction container 103 can be employed as long as the configurations do not conflict therebetween.

### Modification Example 1

In the above-described embodiments, an example in which the nucleic acid amplification reaction device 1 does not include a detection device is illustrated, but as illustrated in Figs. 10A and 10B, the nucleic acid amplification reaction device 2 according to the modification example may include a fluorescence detector 40 for measuring the nucleic acid amount via the side wall of the second region 112 of the reaction container 100. Accordingly, for example, the nucleic acid amplification reaction device 2 can be used for causing fluorescence detection similar to real time PCR. The number of fluorescence detectors 40 is arbitrary as long as the detection can be performed without a problem. In the modification example, the fluorescence detection is performed as one fluorescence detector 40 moves along a slide 22. In order to perform the fluorescence detection, a hole is provided in a side surface portion on the second heating portion 13 side of the main body 10a, and a measuring window 18 (refer to Fig. 11) is formed. When the reaction liquid 140 is positioned in the second region 112, the fluorescence detector 40 irradiate the side wall of the second region 112 of the reaction container 100 with excitation light through the measuring window 18, the radiated fluorescence is measured, and the nucleic acid amplification amount in the reaction liquid 140 can be measured.

In the modification example, in the nucleic acid amplification reaction device 2 illustrated in Figs. 10A, 10B, and 11, the first heating portion 12 is provided on the lid 50 side, and the second heating portion 13 is provided on the side far from the lid 50. In other words, the positional relationship of the first heating portion 12, the second heating portion 13, and other members included in the main body 10, becomes different from that of the nucleic acid amplification reaction device 1. Except that the positional relationship becomes different, the functions of the first heating portion 12 and the second heating portion 13 are similar to those of the above-described nucleic acid amplification reaction device 1. In the modification example, as illustrated in Fig. 11, the measuring window 18 is provided on the side surface of the second heating portion 13. Accordingly, appropriate fluorescence measurement can be performed in the real time PCR in which the fluorescence measurement is performed on a low temperature side (temperature at which the annealing and the extension reaction are performed).

### Modification Example 2

In the embodiment, the first temperature and the second temperature are constant from the start to the end of the heat cycle, but at least one of the first temperature and the second temperature may be changed in the middle of the processing. The first temperature and the second temperature can be changed by the control of the control portion. By switching the disposition of the first heating portion 12 and the mounting portion 11, and moving the reaction liquid 140, the reaction liquid 140 can be heated to the changed temperature. Therefore, the number of heating portions does not increase, and the structure of the device becomes complicated, and for example, it is possible to perform the reaction which requires combination of two or more types of temperatures similar to the reverse transfer PCR.

### Modification Example 3

In the above-described embodiments, an example in which the mounting portion 11 has a slot structure is illustrated, but the mounting portion 11 may have a structure in which the reaction container 100 can be held. For example, a structure in which the reaction container 100 is embedded in a hollow which corresponds to the shape of the reaction container 100, or a structure in which the reaction container 100 is held to being nipped, may be employed.

### Modification Example 4

In the above-described embodiments, the structure in which the position of the reaction container 100 is determined is the bottom plate 17, but the structure which determines the position may be capable of holding the reaction container 100 at the predetermined position. The structure which determine the position may be a structure provided in the nucleic acid amplification reaction device 2, may be a structure provided in the reaction container 100, or may be a structure obtained by combining both two structures. For example, a screw, an inserting type rod, a structure provided with a protruding portion in the reaction container 100, and a structure in which the mounting portion 11 and the reaction container 100 are fitted to each other, can be employed. In a case where the screw or the rod is used, by changing the length of the screw or the insertion length, and the inserting position of the rod, the holding position can be adjusted in accordance with the reaction condition of the heat cycle or the size of the reaction container 100.

### Modification Example 5

In the above-described embodiments, an example in which both the first heating portion 12 and the second heating portion 13 are cartridge heaters is illustrated, but the first heating portion 12 may be a portion which can heat the first region 111 to the first temperature. The second heating portion 13 may be a portion which can heat the second region 112 to the second temperature. For example, as the first heating portion 12 and the second heating portion 13, a carbon heater, a sheet heater, electromagnetic induction heating (IH), a Peltier element, a heating liquid, and heating gas, can be used. In addition, the heating mechanisms which are different in the first heating portion 12 and the second heating portion 13, may employed.

### Modification Example 6

In the above-described embodiments, an example in which the reaction container 100 is heated by the first heating portion 12 and the second heating portion 13 is illustrated, but instead of the second heating portion 13, a cooling portion which cools the second region 112 may be provided. As the cooling portion, for example, the Peltier element can be used. Accordingly, for example, even in a case where the temperature of the second region 112 is unlikely to deteriorate due to the heat from the first region 111 of the reaction container 100, the predetermined temperature gradient can be formed in the flow path 110. In addition, for example, the heat cycle which repeats the heating and cooling can be performed in the reaction liquid 140.

### Modification Example 7

In the above-described embodiments, an example in which the material of the first heat block 12b and the second heat block 13b is aluminum is illustrated, but the material of a heat block can be selected considering conditions, such as thermal conductivity, heat retaining properties, and ease of processing. For example, a copper alloy may be used, or a plurality of materials may be combined with each other. In addition, the first heat block 12b and the second heat block 13b may be made of different materials.

### Modification Example 8

As described in the examples of the above-described embodiments, in a case where the mounting portion 11 is formed as a part of the first heating portion 12, the mounting portion 11 may be provided with a mechanism which is tightly adhered to the reaction container 100. The tightly adhered mechanism may be capable of tightly adhering at least a part of the reaction container 100 to the mounting portion 11. For example, the reaction container 100 may be pressed to one wall surface of the mounting portion 11 by a spring provided in the main body 10 or the lid 50. Accordingly, since the heat of the first heating portion 12 can be further stabilized and transmitted to the nucleic acid amplification reaction container 100, the temperature of the reaction container 100 can be further stabilized.

In addition, in the above-described embodiments, the reaction container 100 is described as a solid (rigid) container which is unlikely to be deformed, but the reaction container 100 may have flexibility. For example, at least one of the first wall 118 and the second wall 119 may be formed to be thin, and may be formed of a soft material. In this manner, in a case where the reaction container 100 is mounted on the nucleic acid amplification reaction device 1, and is heated, and in a case where the reaction liquid 140 or the gas 130 in the flow path 110 is heated and the internal pressure increases, at least a part of the reaction container 100 can be tightly adhered to the mounting portion 11. In other words, in a case where the inner pressure increases, the force which makes the reaction container 100 swollen is suppressed by the mounting portion 11, and thus, both the reaction container 100 and the mounting portion 11 are likely to be tightly adhered. Accordingly, since the heat of the first heating portion 12 can be further stabilized and transmitted to the nucleic acid amplification reaction container 100, the temperature of the reaction container 100 can be further stabilized. In addition, it is also possible to contribute to preventing the reaction container 100 from being detached.

The first wall 118 and the second wall 119 having flexibility can be employed even in the above-described reaction container 101, the reaction container 102, and the reaction container 103, and in particular, since at least one of the first wall 118 and the second wall 119 is formed by the film portion 124, it is possible to easily employ the first wall 118 or the second wall 119 in the reaction container 102 and the reaction container 103. In addition, the thickness of the first wall 118 and the second wall 119 for making the first wall 118 and the second wall 119 flexible depends on the material, but, for example, the thickness is from 0.01 mm to 0.4 mm, preferably from 0.05 mm to 0.3 mm, and more preferably from 0.05 mm to 0.2 mm.

### Modification Example 9

In the above-described embodiments, an example in which the temperature of the first heating portion 12 and the second heating portion 13 is controlled to be substantially equivalent to the temperature obtained by heating the reaction container 100 is illustrated, but the temperature control of the first heating portion 12 and the second heating portion 13 is not limited to the embodiments. The temperature of the first heating portion 12 and the second heating portion 13 may be controlled so that the first region 111 and the second region 112 of the reaction container 100 are heated to the predetermined temperature. For example, considering the material or the size of the reaction container 100, the temperature of the first region 111 and the second region 112 can be more accurately heated to the predetermined temperature.

### Modification Example 10

In the above-described embodiments, an example in which the driving mechanism 20 is a motor is illustrated, but the driving mechanism 20 may be a mechanism which can drive the mounting portion 11, the first heating portion 12, and the second heating portion 13. In a case where the driving mechanism 20 is a mechanism of rotating the mounting portion 11, the first heating portion 12, and the second heating portion 13, it is preferable that the driving mechanism 20 can perform the control so that the rotating speed does not become equal to or greater than the rotating speed by which the reaction liquid 140 does not drop due to a centrifugal force. In addition, in order to eliminate the torsion generated in the wiring, it is preferable that the rotational direction can be reversed. As the mechanism, for example, a handle or a spiral spring can be employed.

### Modification Example 11

In the above-described embodiments, an example in which the mounting portion 11 is a part of the first heating portion 12 is illustrated, but the mounting portion 11 and the first heating portion 12 may be different members as long as the positional relationship of the mounting portion 11 and the first heating portion 12 does not change in a case where the driving mechanism 20 is operated. In a case where the mounting portion 11 and the first heating portion 12 are different members, it is preferable that both the mounting portion 11 and the first heating portion 12 are fixed directly or via other members. In addition, the mounting portion 11 and the first heating portion 12 may be driven by the same mechanism, the mounting portion 11 and the first heating portion 12 may be driven by an additional mechanism, but it is preferable that the mounting portion 11 and the first heating portion 12 are operated so that the positional relationship thereof is maintained to be constant. Accordingly, since the positional relationship of the mounting portion 11 and the first heating portion 12 can be maintained to be constant in a case where the driving mechanism 20 is operated, it is possible to heat the predetermined region of the reaction container 100 to the predetermined temperature. In addition, in a case where the mechanism which drives the mounting portion 11, the first heating portion 12, and the second heating portion 13 is an additional mechanism, the driving mechanism 20 is achieved by combining both of the mechanisms.

### Modification Example 12

In the above-described embodiments, an example in which a temperature sensor is a thermocouple is illustrated, but, for example, a resistance thermometer or a thermistor may be used.

### Modification Example 13

In the above-described embodiments, an example in which the fixing portion 51 is a magnet is illustrated, but the fixing portion 51 may be a portion which can fix the lid 50 and the main body 10. For example, a hinge or a catch clip may be employed.

### Modification Example 14

In the above-described embodiments, the direction of the driving shaft is perpendicular to the longitudinal direction of the mounting portion 11, but the direction of the driving shaft is arbitrary as long as the disposition of the mounting portion 11, the first heating portion 12, and the second heating portion 13 is switched between the first disposition and the second disposition. In a case where the driving mechanism 20 is a mechanism which rotates and drives the mounting portion 11, the first heating portion 12, and the second heating portion 13, by using the straight line which is not parallel to the longitudinal direction of the mounting portion 11 as a shaft, the disposition of the mounting portion 11, the first heating portion 12, and the second heating portion 13 can be switched.

### Modification Example 15

In the above-described embodiments, an example in which the control portion performs the electronic control is illustrated, but the control portion (time control portion) which controls the first time period or the second time period may be a control portion which can control the first time period or the second time period. In other words, the control portion may be capable of controlling the timing of operation or stop of the driving mechanism 20. In addition, the control portion (cycle number control portion) which controls the cycle number of the heat cycles may be a control portion which can control the cycle number. As the time control portion and the cycle number control portion, for example, a physical mechanism or an electronic control mechanism, and a combination thereof can be employed.

### Modification Example 16

As illustrated in Figs. 10A and 10B, the nucleic acid amplification reaction device may include a setting portion 25. The setting portion 25 is a user interface (UI), and is an instrument which sets the condition of the heat cycle. By operating the setting portion 25, at least one of the first temperature, the second temperature, the first time period, the second time period, and the cycle number of the heat cycles, can be set. The setting portion 25 is mechanically or electronically interlocked with the control portion, and the setting in the setting portion 25 is reflected to the control of the control portion. Accordingly, since the condition of reaction can be changed, the predetermined heat cycle can be performed with respect to the reaction liquid 140. Even when any of the above-described items can be separately set, for example, when the setting portion 25 selects one of the plurality of reaction conditions registered in advance, necessary items may be automatically set. In the example of Figs. 10A and 10B, the setting portion 25 is a button type, and reaction condition can be set by pressing the button for each item.

### Modification Example 17

As illustrated in the examples of Figs. 10A and 10B, the nucleic acid amplification reaction device may include a display portion 24. The display portion 24 is a display device, and displays various types of information related to the nucleic acid amplification reaction device. The display portion 24 may display the condition set by the setting portion 25, or real time or temperature in the middle of the heat cycle processing. For example, the input condition may be displayed in a case where the setting is performed, or the temperature measured by the temperature sensor, the time period which has passed in the first disposition and the second disposition, and the cycle number of performed heat cycles, may be displayed in the middle of the heat cycle processing. In addition, in a case where the heat cycle processing is ended, or in a case where any abnormality is generated in the device, the note about the cases may be displayed. Furthermore, the notification may be performed by the sound. By performing the notification by the display or the sound, the user of the device can easily grasp the progress or the end of the heat cycle processing.

### Modification Example 18

In the above-described embodiments, the gas 130 and the reaction liquid 140 are guided into the reaction container 100. However, oil may be guided into the flow path 110 of the reaction container 100. As the oil, for example, dimethylsilicone oil or paraffin oil can be used, and oil having a smaller specific gravity than that of the reaction liquid 140 is preferable. According to this, since the oil is positioned between the reaction liquid 140 and the gas 130 in the flow path 110, evaporation of the components (moisture or the like) of the reaction liquid 140 is suppressed, and the nucleic acid can be more accurately amplified. In addition, even when the oil is present in the flow path 110, since the gas 130 is present, the moving speed of the reaction liquid 140 is unlikely to deteriorate. In addition, even in this case, as described in the above-described embodiments, it is preferable that the ratio of the volume of the gas 130 is 50% or more of the capacity of the flow path 110.

### Modification Example 19

In the above-described embodiments, the direction of rotation in step S104 and the direction of the rotation in step S107 are opposite to each other, but after the rotation in the same direction is performed plural times, the rotation may be performed the same number of times in the opposite direction. Accordingly, since the torsion generated in the wiring can be eliminated, compared to a case where the rotation in the opposite direction is not performed, deterioration of the wiring can be suppressed.

### Modification Example 20

The nucleic acid amplification reaction device 1 in the above-described embodiments includes the first heating portion 12 and the second heating portion 13, but the second heating portion 13 may not be provided. In other words, only the first heating portion 12 may be provided as the heating portion. Accordingly, since the number of members to be used can be reduced, the manufacturing cost can be reduced.

In addition, in the modification example, by heating the first region 111 of the reaction container 100 by the first heating portion 12, the temperature gradient is formed in the reaction container 100 in which the temperature decreases as the distance from the first region 111 increases. Since the second region 112 is a region different from the first region 111, the second temperature which is lower than the temperature of the first region 111 is maintained. In the modification example, the second temperature is controlled, for example, according to the design of the reaction container 100, properties of the gas 130, and the setting of the temperature of the first heating portion 12.

Furthermore, in the modification example, by switching the disposition of the mounting portion 11 and the first heating portion 12 between the first disposition and the second disposition by the driving mechanism 20, the reaction liquid 140 can be moved between the first region 111 and the second region 112. Since the first region 111 and the second region 112 are maintained at different temperature, the heat cycle can be performed with respect to the reaction liquid 140.

In this manner, in a case where the second heating portion 13 is not provided, the spacer 14 holds the first heating portion 12. Accordingly, since the position of the first heating portion 12 can be more accurately determined in the main body 10, it is possible to more reliably heat the first region 111. In a case where the spacer 14 is a heat insulating material, by disposing the spacer 14 to surround the region of the reaction container 100 except for the region heated by the first heating portion 12, the temperature of the first region 111 and the second region 112 can be further stabilized.

The nucleic acid amplification reaction device of the modification example may have a mechanism which maintains the temperature of the main body 10 to be constant. Accordingly, since the temperature of the second region 112 of the reaction container 100 is further stabilized, more accurate heat cycle can be performed with respect to the reaction liquid 140. As the mechanism which maintains the temperature of the main body 10, for example, a thermostatic tank can be used.

### Modification Example 21

In the above-described embodiments, an example in which the nucleic acid amplification reaction device 1 includes the lid 50 is illustrated, but the lid 50 may not be provided. Accordingly, since the number of members to be used can be reduced, the manufacturing cost can be reduced.

### Modification Example 22

In the above-described embodiments, an example in which the nucleic acid amplification reaction device 1 includes the spacer 14 is illustrated, but the spacer 14 may not be provided. For example, instead of the spacer 14 in the above-described embodiments, the space taken by the spacer 14 may be filled with the air. Accordingly, there is a case where the heat conduction between the heaters can be further reduced. In addition, since the number of members to be used can be reduced, the manufacturing cost can be reduced.

### Modification Example 23

In the above-described embodiments, an example in which the nucleic acid amplification reaction device 1 includes the bottom plate 17 is illustrated, but as illustrated in Fig. 11, the bottom plate 17 may not be provided. Accordingly, since the number of members to be used can be reduced, the manufacturing costs can be reduced.

### Modification Example 24

In the above-described embodiments, an example in which the nucleic acid amplification reaction device 1 includes the fixing plate 19 is illustrated, but the fixing plate 19 may not be provided. Accordingly, since the number of members to be used can be reduced, the manufacturing cost can be reduced.

### Modification Example 25

In the above-described embodiments, an example in which the spacer 14 and the fixing plate 19 are separated members is illustrated, but as illustrated in Fig. 11, the spacer 14 and the fixing plate 19 may be integrally formed. In addition, the bottom plate 17 and the spacer 14, or the bottom plate 17 and the fixing plate 19 may be integrally formed.

### Modification Example 26

The spacer 14 and the fixing plate 19 may be transparent. Accordingly, in a case where the transparent reaction container 100 is used in the heat cycle processing, a state where the reaction liquid 140 moves can be observed from the outside of the device. Therefore, it can be visually confirmed whether or not the heat cycle processing can be appropriately performed. Therefore, the degree of "transparency" may be a degree by which the reaction liquid 140 can be visually confirmed in a case where the members are employed in the nucleic acid amplification reaction device 1, and the heat cycle processing is performed.

### Modification Example 27

In order to observe the inside of the nucleic acid amplification reaction device 1, the spacer 14 may be transparent and the fixing plate 19 may not be provided, the fixing plate 19 may be transparent and the spacer 14 may not be provided, and both of the spacer 14 and the fixing plate 19 may not be provided. As the size of the member interposed between the observer and the reaction container 100 which is a target to be observed decreases, the influence of light refraction by the object decreases, and thus, the observation of the inside becomes easy. In addition, when the member is small, the manufacturing cost can be reduced.

### Modification Example 28

In order to observe the inside of the nucleic acid amplification reaction device 1, as illustrated in the examples of Figs. 10A, 10B, and 11, an observation window 23 may be provided in the main body 10a. The observation window 23 may be, for example, a hole or a slit formed in the spacer 14 or the fixing plate 19. In the example of Fig. 11, the observation window 23 is a recessed portion provided in the transparent spacer 14 formed to be integrated with the fixing plate 19. By providing the observation window 23, the thickness of the member which is present between the observer and the reaction container 100 which is a target to be observed can be reduced, and thus, the observation of the inside becomes easy.

### Modification Example 29

In the above-described embodiments, an example in which the first heating portion 12 is disposed on the bottom plate 17 side of the main body 10, and the second heating portion 13 is disposed on the lid 50 side, but, as illustrated in Fig. 11, the first heating portion 12 may be disposed on the lid 50 side. In a case where the first heating portion 12 is disposed on the lid 50 side, the disposition of the mounting portion 11, the first heating portion 12 , and the second heating portion 13 in a case where the reaction container 100 is mounted in step S101 of the above-described embodiments is the second disposition. In other words, the second region 112 is disposed to be positioned in the lowest portion of the flow path 110 in the direction in which the gravity acts. Therefore, in a case where the nucleic acid amplification reaction device 2 of the modification example is employed in the heat cycle processing according to the embodiment, when the reaction container 100 is mounted on the mounting portion 11, the disposition is switched to the first disposition. Specifically, before the process moves to step S102 and step S103 from step S101, the processing of step S107 is performed.

### Modification Example 30

In the above-described embodiments, an example in which the heating the nucleic acid amplification reaction container 100 by the first heating portion 12 and the second heating portion 13 (step S102), and the determining whether or not the first time period has passed (step S103) are started when the reaction container 100 is mounted on the mounting portion 11 (step S101) is illustrated, but the timing of starting step S102 is not limited to the above-described embodiments. Step S102 may be started at an arbitrary timing as long as the first region 111 is heated to the first temperature until the timing is started in step S103. The timing at which step S102 is performed is decided considering the size or the material of the reaction container 100 to be used, or the time necessary for heating the first heat block 12b. For example, before step S101, at the same time of step S101, and after step S101 and before step S103, step S102 may be performed.

### Modification Example 31

In the embodiments, an example in which the first temperature, the second temperature, the first time period, the second time period, the cycle number of heat cycles, and the operation of the driving mechanism 20, are controlled by the control portion is illustrated, but the user can control at least one of the items. In a case where the user controls the first temperature or the second temperature, for example, the temperature measured by the temperature sensor is displayed on the display portion 24, and the user may operate the setting portion 25 and adjust the temperature. In a case where the user controls the cycle number of heat cycles, the user stops the nucleic acid amplification reaction device 1 in a case where the cycle number reaches the predetermined number. Even when the user counts the cycle number, the nucleic acid amplification reaction device 1 may count the cycle number and display the counted cycle number on the display portion 24.

In a case where the user controls the first time period or the second time period, the user determines whether or not the time period reaches the predetermined time period, and switches the disposition of the mounting portion 11, the first heating portion 12, and the second heating portion 13 in the nucleic acid amplification reaction device 2. In other words, the user performs at least a part of step S103 and step S105, and step S104 and step S107 of Fig. 9. Even when the time period is measured by using a timer which is not interlocked with the nucleic acid amplification reaction device 2, passed time period may be displayed on the display portion 24 of the nucleic acid amplification reaction device 2. Even when operating the setting portion 25 (UI), the switching of the disposition may be manually performed by employing a handle to the driving mechanism 20.

### Modification Example 32

In the above-described embodiments, an example in which a rotation angle in a case where the disposition of the mounting portion 11, the first heating portion 12, and the second heating portion 13 is switched by the rotation of the driving mechanism 20 is 180° is illustrated, but the rotation angle may be an angle by which the vertical positional relationship between the first region 111 and the second region 112 changes in the gravity direction. For example, when the rotation angle is less than 180°, the moving speed of the reaction liquid 140 becomes slow. Therefore, by adjusting the rotation angle, it is possible to adjust the time period during which the reaction liquid 140 moves between the first temperature and the second temperature. In other words, it is possible to adjust the time period during which the temperature of the reaction liquid 140 changes between the first temperature and the second temperature.

### Modification Example 33

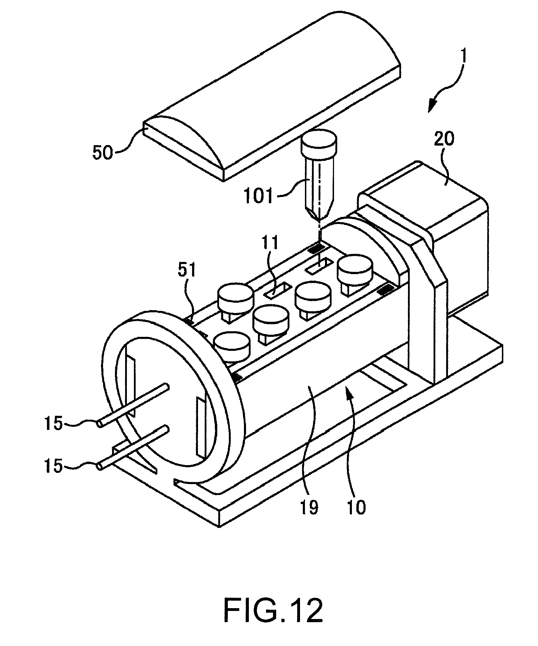

Fig. 12 is a schematic view illustrating a state where the above-described reaction container 101 is mounted on the above-described nucleic acid amplification reaction device 1. As illustrated in Fig. 12, even in a case of the cylindrical reaction container 101 having a cylindrical shape similar to the reaction container 101, it is similarly possible to mount the reaction container 101 on the nucleic acid amplification reaction device 1, and to perform the PCR. In this case, the user can grasp a cylindrical part of the reaction container 101, and workability can be improved in some cases. In addition, although not illustrated, it is similarly easy to mount the reaction container 102 and the reaction container 103 on the nucleic acid amplification reaction device 1.

### Modification Example 34

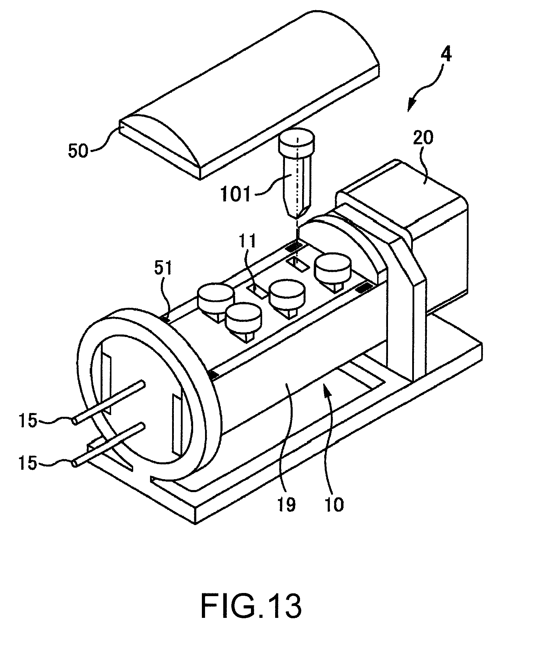

Fig. 13 is a perspective view of a nucleic acid amplification reaction device 3 according to the modification example. Fig. 13 illustrates a state where the lid 50 is open. The nucleic acid amplification reaction device 3 according to the modification example can be obtained by arbitrarily combining the configurations of the above-described embodiments and the modification examples as long as the configurations do not conflict therebetween.

The nucleic acid amplification reaction device 3 is different from the above-described nucleic acid amplification reaction device 1 and the nucleic acid amplification reaction device 2 in the orientation of the mounting portion 11. Except for the difference, the nucleic acid amplification reaction device 3 is similar to the above-described nucleic acid amplification reaction device 1 and the nucleic acid amplification reaction device 2, configuration elements similar to those of the embodiments will be given the same reference numerals, and the description thereof will be omitted.

In any of the nucleic acid amplification reaction device 1 and the nucleic acid amplification reaction device 2, the direction in which the rotating shaft of the driving mechanism 20 extends is parallel to the first inner wall surface 113 and the second inner wall surface 114 of the reaction container. The nucleic acid amplification reaction device 3 is perpendicular to the first inner wall surface 113 and the second inner wall surface 114 of the reaction container. Even when the direction in which the rotating shaft of the driving mechanism 20 extends is parallel to or perpendicular to the first inner wall surface 113 and the second inner wall surface 114 of the reaction container, by reversing the reaction container, the reaction liquid 140 in the flow path 110 can move by the action of the gravity. However, the flow path 110 of the reaction container becomes flatter, for example, in the section of the flow path 110 orthogonal to the longitudinal direction, in a case where magnification (aspect ratio) with respect to the length in the direction parallel to the first inner wall surface 113, of the distance between the first inner wall surface 113 and the second inner wall surface 114 (for example, 7 times or more), similar to the nucleic acid amplification reaction device 3, as being perpendicular to the first inner wall surface 113 and the second inner wall surface 114 of the reaction container, there is a case where the movement of the reaction liquid 140 can be smoothly performed. It is considered that this is because, on the interface between the reaction liquid 140 and the gas 130, a period which becomes longer is generated along the direction in which the gravity acts in the middle of the rotation.

### 5. Experiment Example

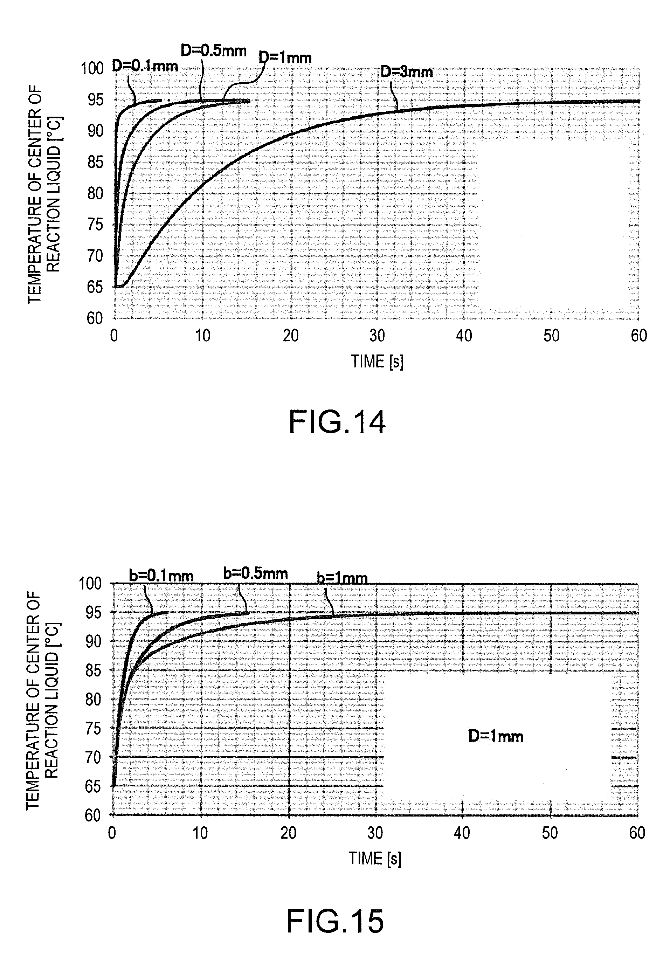

A result of investigation by simulating (computer experiment) the influence of the shape of the nucleic acid amplification reaction container according to the invention on the change in temperature of the reaction liquid, will be described. Fig. 14 is a graph illustrating the influence of the distance between the first inner wall surface and the second inner wall surface of the nucleic acid amplification reaction container on the change in temperature of the reaction liquid. Fig. 15 is a graph illustrating the influence of the thickness of the first wall and the second wall of the nucleic acid amplification reaction container on the change in temperature of the reaction liquid.

In the computer experiment, the reaction liquid was disposed between two parallel flat plates (correspond to the first wall 118 and the second wall 119 of the above-described embodiments) which are disposed to be separated from each other by an interval D (corresponds to the width a in Figs. 4A and 4B) and has the thickness of 0.5 mm, the initial temperature was set in each region and the boundary condition was set on the outside of the flat plates, and temporal transition of the temperature of the center part of the reaction liquid was simulated. The initial condition was set so that the initial temperature of the flat plate was 95°C and the initial temperature of the reaction liquid was 65°C, and the boundary condition was set so that the computation was performed by changing the interval D while the temperature on the outside of the flat plate was constant to be 95°C.

Accordingly, for example, when the interval D is 0.1 mm, the contact areas of the reaction liquid with respect to each plane respectively become 200 mm² (2 cm²), and when the interval D is 1 mm, the contact areas of the reaction liquid with respect to each plane respectively become 20 mm².

Fig. 14 illustrates the transition of the temperature of the center portion of the reaction liquid when the interval D changes. In Fig. 14, as the interval D decreases, the time period during which the reaction liquid achieves approximately 95°C decreases. For example, in a case where the interval D is 0.1 mm, the reaction liquid achieves approximately 95°C in approximately 5 seconds. In addition, it is ascertained that, in a case where the interval D is 0.5 mm and 1 mm, the reaction liquid achieves approximately 95°C in approximately 15 seconds, and even when the interval D is 3 mm, the reaction liquid achieves approximately 95°C within 1 minute.

Fig. 15 illustrates a simulation in which a material (corresponds to the first wall 118 and the second wall 119 of the above-described embodiments) which has the constant interval D of the parallel planes of 1 mm, thermal conductivity on the outside of the plane of 0.12 (W/m·K), and the thickness b was disposed, and the temporal transition of the temperature of the center part of the reaction liquid was simulated. The initial condition was set so that the initial temperature of the flat plate was 95°C and the initial temperature of the reaction liquid was 65°C, and the boundary condition was set so that the temperature on the outside of the flat plate was 95°C.

In Fig. 15, as the thickness b decreases, the time period during which the reaction liquid achieves approximately 95°C decreases. For example, in a case where the thickness b is 0.1 mm, the reaction liquid achieves approximately 95°C in approximately 5 seconds. In addition, it is ascertained that, in a case where the thickness b is 0.5 mm, the reaction liquid achieves approximately 95°C in approximately 15 seconds, and even when the thickness b is 1 mm, the reaction liquid achieves approximately 95°C within 1 minute.

The invention is not limited to the above-described embodiments, and more various modifications are possible. For example, the invention includes configurations (for example, configurations having the same functions, methods, and the results, or configurations having the same purpose and effects) which are practically the same as the configurations described in the embodiments. In addition, the invention includes configurations in which parts which are not essential of the configurations described in the embodiments are replaced. In addition, the invention includes configurations in which action effects which are the same as those of the configurations described in the embodiments, or configurations which can achieve the same purpose as those of the configurations described in the embodiments. In addition, the invention includes configurations obtained by adding the known technology to the configurations described in the embodiments.

## Claims

1. A nucleic acid amplification reaction container comprising:
a flow path which is formed between a first inner wall surface and a second inner wall surface that opposes the first inner wall surface, and has the longitudinal direction in the direction along the first inner wall surface,
wherein the distance between the first inner wall surface and the second inner wall surface is the distance by which nucleic acid amplification reaction liquid comes into contact with both of the first inner wall surface and the second inner wall surface in a case where the nucleic acid amplification reaction liquid is guided therein, and
wherein, in the section which is orthogonal to the longitudinal direction of the flow path, the distance in the direction parallel to the first inner wall surface is 5 times or more the distance between the first inner wall surface and the second inner wall surface.

2. A nucleic acid amplification reaction container comprising:
a flow path which is formed between a first inner wall surface and a second inner wall surface that opposes the first inner wall surface, and has the longitudinal direction in a case of being seen from the direction perpendicular to the first inner wall surface,
wherein the distance between the first inner wall surface and the second inner wall surface is the distance by which nucleic acid amplification reaction liquid comes into contact with both of the first inner wall surface and the second inner wall surface in a case where the nucleic acid amplification reaction liquid is guided therein, and
wherein the nucleic acid amplification reaction liquid and gas are disposed in the flow path.

3. The nucleic acid amplification reaction container according to claim 1 or claim 2,
wherein oil is disposed in the flow path.

4. The nucleic acid amplification reaction container according to claim 2 or claim 3,
wherein the volume of the gas is 50% or more of the capacity of the flow path.

5. The nucleic acid amplification reaction container according to any of claims 1-4,
wherein the distance between the first inner wall surface and the second inner wall surface is from 0.2 mm to 3.0 mm.

6. The nucleic acid amplification reaction container according to any of claims 1-5,
wherein the distance between the first inner wall surface and the second inner wall surface is from 0.2 mm to 3.0 mm.

7. The nucleic acid amplification reaction container according to any of claims 1-6,
wherein at least one of a first wall which forms the first inner wall surface and a second wall which forms the second inner wall surface has a thickness of 0.01 mm to 0.5 mm.

8. The nucleic acid amplification reaction container according to any of claims 1-7,
wherein at least one of a first wall which forms the first inner wall surface and a second wall which forms the second inner wall surface has a thickness of 0.01 mm to 0.5 mm.

9. The nucleic acid amplification reaction container according to any of claims 1-8,
wherein at least one of the first wall and the second wall is capable of being deformed in a case where inner pressure of the flow path increases.

10. The nucleic acid amplification reaction container according to any of claims 1-9,
wherein at least one of the first wall and the second wall is capable of being deformed in a case where inner pressure of the flow path increases.

11. The nucleic acid amplification reaction container according to any of claims 1-10,
wherein at least one of walls which form the flow path has light transmission properties.

12. The nucleic acid amplification reaction container according to any of claims 1-11,
wherein the volume of the nucleic acid amplification reaction liquid is 1 µl to 100 µl.

13. The nucleic acid amplification reaction container according to any of claims 1-12,
wherein the first inner wall surface and the second inner wall surface are hydrophobic.

14. The nucleic acid amplification reaction container according to any of claims 1-13, further comprising:
a lid body which seals the flow path,
wherein the lid body has a shape including a cylindrical surface which considers the longitudinal direction as a shaft.

15. A nucleic acid amplification reaction device comprising:
the nucleic acid amplification reaction container according to any of claims 1-14;
a mounting portion which mounts the nucleic acid amplification reaction container;
a first heating portion which heats a first region of the flow path to a first temperature, in a case where the nucleic acid amplification reaction container is mounted on the mounting portion;
a second heating portion which heats a second region different from the first region of the flow path to a second temperature different from the first temperature, in a case where the nucleic acid amplification reaction container is mounted on the mounting portion; and
a driving mechanism which switches disposition of the mounting portion, the first region, and the second region between a first disposition and a second disposition, in a case where the nucleic acid amplification reaction container is mounted on the mounting portion,
wherein the first disposition is a disposition in which the first region is below the second region with respect to the direction in which gravity acts, and
wherein the second disposition is a disposition in which the second region is below the first region with respect to the direction in which gravity acts.

16. A reaction method for amplifying nucleic acid comprising:
guiding reaction liquid into the nucleic acid amplification reaction container according to any of claims 1-14, and disposing at least the nucleic acid amplification reaction liquid and gas in the flow path;
heating a first region of the flow path to a first temperature;
heating a second region different from the first region of the flow path to a second temperature different from the first temperature;
moving the reaction liquid in the flow path, and moving the reaction liquid from the first region to the second region; and
moving the reaction liquid in the flow path, and moving the reaction liquid from the second region to the first region.
